# EUROPÄISCHE PATENTSCHRIFT

(11) **EP 2 326 289 B1**
(45) Veröffentlichungstag und Bekanntmachung des Hinweises auf die Patenterteilung: **28.10.2020**
(21) Anmeldenummer: 09771886.0
(22) Anmeldetag: 09.06.2009
(51) Int. Cl.: A61F 2/95

(54) **EINRICHTUNG UND VERFAHREN ZUR BEREITSTELLUNG EINES STENTS ZUR IMPLANTATION**
DEVICE AND METHOD FOR PROVIDING A STENT FOR IMPLANTATION
DISPOSITIF ET PROCÉDÉ DE MISE EN OEUVRE D'UN STENT POUR L'IMPLANTATION

(30) Priorität: 04.07.2008 CH 10362008
(43) Veröffentlichungstag der Anmeldung: 01.06.2011
(73) Patentinhaber: Qvanteq AG, 8005 Zürich (CH)
(72) Erfinder: MÄDER, Armin, W., 8805 Richterswil (CH); ZUCKER, Arik, 8003 Zürich (CH)
(74) Vertreter: Latscha Schöllhorn Partner AG
(86) Internationale Anmeldenummer: PCT/CH2009/000189
(87) Internationale Veröffentlichungsnummer: WO 2010/000079

(56) Entgegenhaltungen:
- WO-A1-2006/086709
- WO-A1-2006/086709
- US-A1- 2003 187 493
- US-A1- 2003 187 493
- US-A1- 2006 183 383
- US-A1- 2006 183 383
- US-A1- 2007 288 085
- US-A1- 2007 288 085
- US-A1- 2008 086 198
- US-A1- 2008 086 198

## Beschreibung

### TECHNISCHES GEBIET

Die vorliegende Erfindung betrifft eine Einrichtung zur Bereitstellung eines Stents zur Implantation in ein Körperlumen, insbesondere zum Komprimieren eines ballon-expandierenden Stents auf einen Ballonkatheter oder eines selbst-expandierenden Stents zum Einführen in einen Schlauchkatheter, und ein Verfahren zur Bereitstellung des Stents für eine Implantation.

### STAND DER TECHNIK

Stents werden z. B. als medizinisches Implantat zur Behandlung von Läsionen in Blutgefässen verwendet. Ein Stent hat im Allgemeinen eine Vielzahl von Stegen, die miteinander eine Röhrenform bilden. Zwischen einem proximalen und einem distalen Ende erstrecken sich die Stentlänge und als Durchgang das Stentlumen mit einem komprimierbaren Durchmesser. Im dilatierten bzw. freigesetzten Zustand nimmt der Stent einen expandierten Durchmesser z. B. zum Stützen des Blutgefässes ein. Zur Begünstigung der Hämokompatibilität kann die Stentoberfläche hydrophil ausgebildet sein.

Ein spezieller Anwendungsbereich ist die Gefässdilatation im Gebiet der perkutanen transluminalen Angioplastie, darunter auch kardiovaskuläre Intervention. Derartige Stents werden mit einem speziell dafür vorgesehenen Katheter durch eine minimale Öffnung, z.B. mittels Arterienpunktion im Oberschenkelbereich in den menschlichen Körper bis zur Läsion, d.h. der zu behandelnden Gefässverengung, eingeführt und dort dilatiert. Während der Stent im dilatierten Blutgefäss verbleibt und dieses von innen her stützt, wird der Katheter aus dem Körper entfernt. Der Blutfluss ist durch das dilatierte und unterstützte Gefäss wieder gewährleistet. Dieser Vorgang wird mit Hilfe von instantanen Röntgenaufnahmen durchgeführt, welche sowohl die Blutgefässe, als auch die in den Körper eingeführten Instrumente auf einem Monitor darstellen.

Ein anderer spezieller Anwendungsbereich ist die Behandlung von Aneurysmen, d.h. erweiterten Blutgefässen. Bei dieser Behandlung wird ein Stent Graft - bestehend aus einem Stützgeflecht und einer Ummantelung - in das Aneurysma eingesetzt, um den herkömmlichen Blutfluss wieder zu gewährleisten. Ferner kann ein Stent auch weitere funktionelle Elemente umfassen, wie etwa Verschlusselemente zum Verschluss eines Lumens, Klappenersatzelemente, etc., wie es aus dem Stand der Technik bekannt ist.

Stents sind zudem in einer Vielzahl weiterer medizinischer Anwendungen im Stand der Technik weit verbreitet. Im wesentlichen wird zwischen ballon-expandierenden oder selbst-expandierenden Stents unterschieden. Ballon-expandierende Stents werden vor ihrer Implantation auf einen nichtexpandierten Ballon aufgebracht. Hierfür wird beispielsweise der Stent über dem Ballon auf einen kleineren Durchmesser komprimiert und gemeinsam mit dem Ballon in den Körper eingeführt. Am Behandlungsort, z. B. bei einer Läsion oder einer Gefässklappe, wird der Ballon expandiert, so dass er den Stent aufweitet. Anschliessend kann der Ballon aus dem Körper entfernt werden. Selbst-expandierende Stents bestehen beispielsweise aus einem Metall mit Memory-Effekt. Sie können zum Einführen in ein Körperlumen entgegen ihrer Spannkraft komprimiert und in einen Zuführkatheter eingesetzt werden. Am Behandlungsort werden sie aus dem Katheter freigesetzt und springen zurück in ihren expandierten Zustand.

Die in Blutgefässen implantierten metallischen Stents bergen jedoch gewisse Risiken für den Patienten. Unter anderem können sich Thrombosen an den Strukturen des Stents bilden. In Kombination mit Medikamenten, welche man den Patienten nach der Implantation verabreichte, konnten die Fälle von Thrombosen bei "nackten" Metall-Stents (Bare Metal Stents, BMS) unter 1% innerhalb der ersten 10 Tage gesenkt werden. Dennoch ist dies eine der meist gefürchteten Komplikationen, insbesondere bei der Koronarintervention.

Ein von den Medizinern gewünschtes Verhalten der Stents ist ein raschmögliches Einwachsen, die sogenannte Reendothelialisierung. Diese ist äusserst wichtig für den Erfolg der Stent-Therapie, weil die Zellen in dieser Endothelschicht essentielle antithrombogene Faktoren bilden. Solange der Stent jedoch noch nicht eingewachsen ist, und seine Strukturen dem Blutfluss ausgesetzt sind, ist es äusserst wichtig, eine antithrombogene Stentoberfläche bereit zu stellen.

Es ist bekannt, dass Stents mit hydrophilen Oberflächeneigenschaften eine sehr viel höhere Hämokompatibilität, d.h. eine viel tiefere Thrombogenizität, aufweisen. Um die Hydrophilizität auf den Stentoberflächen zu erhöhen, werden beispielsweise mittels Beschichtungsverfahren Substanzen auf die Stentoberfläche aufgetragen.

Mögliche Beschichtungsverfahren sind beispielsweise "chemical vapor deposition" (CVD) oder "physical vapor deposition" (PVD), durch welche Materialien, wie z.B. Polymere oder Metalle in definierten Schichtdicken auf die Stentoberfläche aufgetragen werden. An einem mit Polymer beschichteten BMS ergab sich, dass durch die erhöhten hydrophilen Eigenschaften der Oberfläche die Plättchenbildung von 85% (BMS) auf 20% (Polymer beschichteter BMS) reduziert wurde.

Bei einer weiteren Anwendung wird die Stentoberfläche mit einer aktiven Substanzen beschichtet. Als aktive Substanzen werden z. B. Glucocorticoiden, Zytostatika, Immunmodulatoren oder Antiproliferativa eingesetzt. Die Substanzen, und somit der medizinische Wirkstoff, werden nach dem Implantieren des Stents in den Körper sukzessive abgegeben.

Allen Stents gemeinsam ist es, dass sie zum Einführen in ein Körperlumen einen geringeren Durchmesser aufweisen müssen, als bei der Ausübung ihrer Funktion im Köper. In der Regel werden die Stents vom Hersteller auf einen Katheter vormontiert und verpackt. Es ist aber auch möglich, einen Stent erst kurz vor dem Einsetzen der Stents in das Körperlumen zu komprimieren. Bei herkömmlichen Verfahren zur Bereitstellung eines Stents zur Implantation wird der Stent meist in expandiertem oder semi-expandiertem Zustand der erforderlichen Oberflächenbehandlung unterzogen und anschliessend durch eine Crimpvorrichtung auf einen kleineren Durchmesser komprimiert, der zum Einführen in den Körper eines Patienten geeignet ist.

Aus der US 6,968,607 B2 ist beispielsweise eine Crimpvorrichtung bekannt, die aus mehreren Crimpsegmenten besteht. Die Enden der Crimpsegmente sind an einer Trommel entlang einer Kreisform befestigt und um einen Drehpunkt schwenkbar, der entfernt vom Befestigungspunkt liegt. Durch Drehen der Trommel kann das andere Ende der Crimpsegmente zur Kreismitte hin geschwenkt werden. Die Crimpsegmente bilden in aufgeschwenktem Zustand zwischen sich eine zentrale Öffnung, in die ein Stent eingesetzt werden kann, so dass die Segmente den Stent umfassen. Beim Schwenken der Segmente zur Kreismitte hin, wird die zentrale Öffnung verkleinert und die einzelnen Segmente drücken von allen Seiten gegen den Aussenumfang des Stents, so dass dieser komprimiert wird. Die Trommel kann mittels eines Betätigunghebels gedreht werden. Die Stents werden durch eine Ein- und Ausgabeöffnung der Crimpvorrichtung zugeführt und in gecrimptem Zustand entnommen.

Ähnliche Crimpvorrichtungen z. B. mit einer integrierten Einrichtung zur Temperierung des Stents während dem Crimpvorgang oder z. B. mit einer integrierten Einrichtung, welche den Stent während dem Komprimieren mit einer Umhüllung versieht, sind aus der US 2008/0072653 A1 und der WO 2006/050425 A2 bekannt.

Bei einer anderen Crimpvorichtung nach der US 6,141,855 wird ein Stent von einer Mylar-Folie umfasst. Die Enden der Folie werden durch einen Schlitz in einer festen Platte geführt, so dass die Folie eine Schlaufe mit variablem Durchmesser bildet, innerhalb der der Stent angeordnet ist. Zum Komprimieren des Stents wird an den Enden der Folie gezogen, so dass sich der Durchmesser der Schlaufe verringert und der Stent von der Folie zusammengedrückt wird. Mit dieser Crimpvorrichtung kann der Stent z. B. auf den Ballon eines Ballon-Katheters aufgecrimpt werden.

Noch eine weitere Crimpvorrichtung ist aus der US 2006/183383 A1 bekannt und für beschichtete Stents vorgesehen. Zum Schutz von Crimpelementen der Vorrichtung und der Stentoberfläche wird ein rollbares Folienmaterial in das Innere der Crimpvorrichtung eingebracht, so dass es zwischen dem Stent und den Crimpelementen zu liegen kommt. Um das Folienmaterial in das Innere der Crimpvorrichtung einzubringen, muss eines der Crimpelemente entfernt werden, sodass eine seitliche Öffnung entsteht. Durch die Öffnung wird das Folienmaterial mit einem Gebläse eingeblasen, wobei ein für das Folienmaterial inertes Gas verwendet wird. Anschliessend kann der Stent in das Innere der Crimpvorrichtung eingelegt werden. Das Gebläse wird abgestellt, die Öffnung mit dem Crimpelement wieder verschlossen und der Stent komprimiert. Sobald das Gebläse abgestellt wird, kann das Gas durch die Öffnung und an den Enden der Crimpvorrichtung entweichen, so dass der Stent der Umgebung ausgesetzt ist und die Gefahr der Kontamination besteht.

Beim Bereitstellen eines Stents für die Implantation mit Crimpvorrichtungen aus dem Stand der Technik werden die Stents im Allgemeinen beim Einführen in die Crimpvorrichtung ungeschützt der Umgebungsatmosphäre ausgesetzt und von den Elementen der Crimpvorrichtung berührt. Sie unterliegen dabei einer Kontamination z. B. durch in der Luft befindliche Reaktionsstoffe, wie etwa Kohlenwasserstoffemoleküle, die eine hydrophile Oberfläche eines Stents oder eine aktive Substanz auf dem Stent beeinträchtigen können. Auch kann die Stentoberfläche durch Rückstände an den Elementen der Crimpvorrichtung kontaminiert werden. Ferner besteht beim Überführen eines gecrimpten Stents aus einer herkömmlichen Crimpvorrichtung die Gefahr der unerwünschten Kontamination oder Veränderung der Stentoberfläche.

### AUFGABE DER ERFINDUNG

Es ist eine Aufgabe der vorliegenden Erfindung eine Einrichtung und ein Verfahren zur Bereitstellung eines Stents für eine Implantation in ein Körpervolumen vorzuschlagen, welche eine Beeinträchtigung oder Verunreinigung des Stents, insbesondere der Stentoberfläche, während des Komprimierens des Stents verhindern, die Handhabung des Stents bei der Vorbereitung für die Implantation vereinfachen und die Sicherheit gegen unerwünschte Interaktionen der Stentoberfläche vor der Implantation erhöhen. Ferner soll eine Verpackung für die Lagerung und den Transport von zunächst ungecrimpten Stents gezeigt werden, die für den Einsatz in Mitteln für die Montage des Stent auf einen Katheter geeignet sind, wobei insbesondere eine hydrophile Eigenschaft der Stentoberfläche erhalten bleibt.

### DARSTELLUNG DER ERFINDUNG

Diese Aufgabe wird von der Erfindung durch eine Einrichtung und ein Verfahren nach den Ansprüchen 1 und 11 gelöst. Vorteilhafte Ausgestaltungen und verschiedene Ausführungsbeispiele sind in den abhängigen Ansprüchen beschrieben.

Gemäss der vorliegenden Erfindung wird eine Einrichtung zur Bereitstellung eines Stents zur Implantation in ein Körperlumen vorgesehen. Die Einrichtung ist für einen Stent vorgesehen, der ein proximales Ende und ein distales Ende aufweist, wobei sich zwischen den Enden ein Stentlumen mit komprimierbarem Durchmesser erstreckt. Die Einrichtung umfasst eine Crimpvorrichtung mit Elementen, die um eine Achse angeordnet sind und zumindest zum Teil relativ zu einander radial zur Achse beweglich sind, wodurch sich der freie Raum, den die Elemente einschliessen, verkleinert. Weiter umfasst die Einrichtung einen Aktivator zur Betätigung der Crimpvorrichtung. Die Elemente der Crimpvorrichtung umfassen den Stent und sind mittels des Aktivators von einer aufgeweiteten Position, in der der Stent ungecrimpt, bzw. unkomprimiert ist, in eine geschlossene Position, in der der Stent im Durchmesser komprimiert ist, radial beweglich. In der aufgeweiteten Position der Elemente weist der freie Raum, den sie umschliessen, mindestens einen so grossen Durchmesser auf, dass der Stent in expandiertem Zustand in diesem Raum untergebracht werden kann. In der geschlossenen Position der Elemente, sind die Elemente soweit radial nach innen bewegt, dass der freie Raum auf einen Durchmesser verringert wird, der dem Durchmesser entspricht, der für einen komprimierten, bzw. gecrimpten, Stent vorgesehen ist.

Die Elemente der Crimpvorrichtung können als schwenkbare Backen oder als Segmente ausgebildet sein, die ringförmig um eine Achse angeordnet sind und in Richtung der Achse beweglich sind. Die Crimpvorrichtung kann aber auch eine Schlaufe als Element aufweisen, die z. B. zusammengezogen werden kann und somit einen Stent komprimiert, der sich in der Schlaufe befindet. Ferner kann ein Schrumpfschlauch als bewegliches Element der Crimpvorrichtung vorgesehen sein, in den der Stent eingeführt wird und der sich anschliessend durch Erwärmen zusammenzieht. Letztlich können auch Fäden am Stent derart angebracht werden, dass sich der Stent komprimiert, wenn an den Fäden gezogen wird. Derartige Crimpvorrichtungen sind aus dem Stand der Technik bekannt.

In der erfindungsgemässen Einrichtung ist ein Lagerraum bestehend aus einem inerten Medium, bzw. mit einer inerten Füllung vorgesehen, der eine Umhüllung bildet, in der der Stent, während die Elemente den Stent umfassen und von der aufgeweiteten Position in die geschlossene Position beweglich sind, die überwiegende Zeit inert gelagert ist. Der Lagerraum ist somit zumindest teilweise in dem freien Raum, den die Elemente der Crimpvorrichtung einschliessen, vorgesehen, kann sich aber auch radial über die Elemente hinaus erstrecken. Somit kann die Crimpvorrichtung vollständig oder teilweise innerhalb des Lagerraums angeordnet sein. Beispielsweise können die beweglichen Elemente der Crimpvorrichtung innerhalb des Lagerraums vorgesehen sein. Auch Teile eines Antriebs für die beweglichen Elemente, wie etwa eine Antriebswelle, können wenigstens zum Teil innerhalb des Lagerraums in der inerten Umgebung untergebracht sein. Alternativ ist es auch möglich, die Crimpvorrichtung vollständig ausserhalb des Lagerraums anzuordnen, so dass sie durch eine Wandung des Lagerraums auf den Stent wirkt. Der Aktivator für die Crimpvorrichtung ist vorzugsweise ausserhalb des Lagerraums vorgesehen. Weiter ist der Lagerraum als an einem Ende geschlossener Schlauch ausgebildet und seitlich komprimierbar.

Nach der Erfindung ist vorzugsweise der gesamte Inhalt des Lagerraums inert und mit der inerten Füllung gefüllt. Die inerte Füllung des Lagerraums kann z. B. durch ein inertes Gas, wie etwa Argon oder Stickstoff, oder durch Wasser, insbesondere Wasser mit WFI-Qualität (water for injection Qualität), oder durch ein Gel gegeben sein. Im Sinne der Erfindung ist ein Medium oder eine Füllung dann als inert anzusehen, wenn die Reinheit oder Sauberkeit der Oberfläche des Stents von dem Medium oder der Füllung nicht verändert oder beeinträchtigt wird. Es erfolgt somit keinerlei Reaktion zwischen dem Stent und dem Medium oder der Füllung. Auch ein Vakuum im Lagerraum soll somit als inerte Füllung verstanden werden. Im Falle von sterilen Oberflächen verbleiben daher die nach der Sterilisation auf der Oberfläche anhaftenden abgetöteten pyrogenen Stoffe auch im inerten Medium, bzw. der inerten Füllung auf der Oberfläche. Im Fall von hydrophilisierten Oberflächen können sich keine neuen Reaktionsstoffe auf der gereinigten, bzw. gesäuberten Oberfläche ablagern, so dass keine Rekontamination möglich ist.

Der Lagerraum bestehend aus einem inerten Medium, bzw. mit einer inerten Füllung, bildet eine Umhüllung für den Stent, bzw. die Oberfläche des Stents. Die Umhüllung kann z. B. auch durch einen Gasstrom hergestellt werden, der den Stent von allen Seiten umströmt, so dass seine Oberfläche innerhalb einer Gaswolke liegt und von dem Gas bedeckt ist. Der Gasstrom kann beispielweise entlang der Achse der Crimpvorrichtung erfolgen und durch den freien Raum zwischen den beweglichen Elementen geleitet werden. Der Lagerraum wird somit durch den Rand des Gasstroms begrenzt. Als Umhüllung kann auch ein Benetzungsfilm auf der Oberfläche des Stents dienen. Der Stent kann z. B. in WFI-Wasser gelagert werden. Bei der Entnahme des Stents aus dem Wasser bleibt ein Film auf der Oberfläche des Stents zurück, so dass diese benetzt bleibt. Durch den Benetzungsfilm ist der Stent inert gelagert. Der Lagerraum wird in diesem Fall durch die Oberfläche des Benetzungsfilms begrenzt.

Als Lagerraum kann aber auch jeder Behälter oder ein formbarer Beutel dienen, dessen Grösse ausreicht, um den unkomprimierten Stent in sich aufzunehmen, und dessen Wandung ausreichend dicht ist, um das inerte Medium im Innenraum zu halten. Der Lagerraum kann z. B. durch einen Behälter gegeben sein, der mit einem inerten Medium gefüllt ist, das schwerer als Luft ist, wie Argon oder Wasser mit WFI-Qualität. In diesem Fall ist es nicht notwendig den Behälter nach oben dicht zu verschliessen, da das schwere Medium nicht von selbst aus dem Behälter entweicht. Der Stent oder auch die Crimpvorrichtung können somit in einfacher Weise in den Behälter eingebracht werden.

In einer Bereitstellungseinrichtung nach der Erfindung ist der Stent in dem Zeitintervall, während die Elemente den Stent umfassen und von der aufgeweiteten Position in die geschlossene Position bewegt werden, die überwiegende Zeit, vorzugsweise während dem gesamten Zeitintervall, inert gelagert. Das Zeitintervall wird durch die Dauer bestimmt, welche die Crimpvorrichtung benötigt, um durch Bewegung der Crimpelemente den Stent von einem unkomprimierten in einen komprimierten Zustand zu bringen. Die Umhüllung des Stents mit dem inerten Medium, bzw. der inerten Füllung, kann während diesem Vorgang über eine Zeitspanne unterbrochen werden, in der keine signifikante Rekontamination der Stentoberfläche erfolgt.

Eine Rekontamination ist nicht signifikant, sofern sie auf der Oberfläche des Stents kein für den klinischen Erfolg relevantes Ausmass annimmt. Die Dauer der Zeitspanne der Unterbrechung, ohne dass eine signifikante Rekontamination eintritt ist u. a. von der Materialart des Stents und der Rauhigkeit dessen Oberfläche abhängig. Beispielsweise kann ein Nitinol-Stent mehrere Minuten ohne eine inerte Umhüllung sein, ohne dass eine relevante Verunreinigung z. B. durch sich ablagernde Kohlenstoffketten eintritt. Ein Stent kann somit einige Minuten an der freien Atmosphäre bleiben, ohne dass eine signifikante Rekontamination eintritt.

Der Stent kann daher beispielsweise ausserhalb der erfindungsgemässen Bereitstellungseinrichtung gereinigt werden, in gereinigtem Zustand inert aufbewahrt werden und aus dieser inerten Aufbewahrung entnommen werden, um ihn in die Bereitstellungseinrichtung einzuführen. In der erfindungsgemässen Bereitstellungseinrichtung kann der Stent in den Lagerraum eingebracht werden, in dem er erneut inert gelagert ist. Die Zeitspanne der Unterbrechung der inerten Umhüllung des Stents von der inerten Aufbewahrung nach der Reinigung bis zur inerten Lagerung in der Bereitstellungseinrichtung sollte dabei so kurz sein, dass keine signifikante Rekontamination erfolgt, wie oben geschildert. Gleiches gilt für die Entnahme des Stents aus der Bereitstellungseinrichtung nach dem Komprimieren des Stents. Grundsätzlich kann das Komprimieren des Stents auch innerhalb der Zeitspanne der Unterbrechung erfolgen, sofern diese so kurz ist, dass keine Rekontamination einsetzt, wie nachfolgend näher erläutert wird.

Der Lagerraum kann fest in der Bereitstellungseinrichtung vorgesehen sein oder herausnehmbar angeordnet sein. So kann der Lagerraum z. B. gemeinsam mit dem Stent in die Einrichtung eingebracht werden, beispielsweise nach der Vorbehandlung der Stentoberfläche im Lagerraum. Beispielsweise wird der Stent im Lagerraum der Einrichtung einer Behandlung zur Reinigung der Oberfläche unterzogen, wobei der Lagerraum innerhalb der Bereitstellungseinrichtung sein kann. Ist für die Implantation z. B. ein metallischer Stent vorgesehen, dessen Stentoberfläche eine hydrophile Eigenschaft besitzen soll, können die aus der Atmosphäre stammenden molekularen chemischen Verunreinigungen, vorrangig Kohlenwasserstoff-Verbindungen, auf der Oberfläche durch eine geeignete Reinigungsbehandlung signifikant reduziert werden, wodurch sich der Kontaktwinkel als Mass der Hydrophilizität eines an der Oberfläche aufsitzenden Wassertropfens im Verhältnis zum Kontaktwinkel vor dieser Behandlung verringert. Der Stent ist im Lagerraum inert gelagert, um eine natürliche Rekontamination aus der Atmosphäre zu verhindern. Sofern die Behandlung ausserhalb der Bereitstellungseinrichtung erfolgt kann der hydrophilisierte Stent mit dem Lagerraum in die Bereitstellungseinrichtung eingesetzt werden. Grundsätzlich ist es auch denkbar, dass eine Reinigungsbehandlung in einem anderen Behälter, vorzugsweise mit inerter Füllung, erfolgt, wie oben beschrieben, und der Stent nach der Behandlung in den Lagerraum der Bereitstellungseinrichtung eingebracht wird, solange beim Transfer keine signifikante Rekontamination erfolgt.

Es ist auch möglich, den Stent nach dem Komprimieren mit Hilfe eines Transfergefässes aus dem inerten Lagerraum der Bereitstellungseinrichtung zu entnehmen, wobei das Transfergefäss selbst eine inerte Füllung aufweisen kann, vorzugsweise die gleiche, wie im Lagerraum vorgesehen ist. Das Transfergefäss kann in den Lagerraum eingeführt werden und den Stent in sich aufnehmen. Anschliessend wird das Transfergefäss mit samt dem Stent aus dem Lagerraum herausgeholt und nimmt den in sich aufgenommenen Stent mit. Dabei bleibt der Stent vom inerten Medium umhüllt. Gleiches gilt für das Einführen des Stents in den Lagerraum der Bereitstellungseinrichtung. Es kann auch ein Sieb oder eine Pinzette als Transportmittel für den Stent verwendet werden.

Bei einer Einrichtung zur Bereitstellung eines Stents zur Implantation in ein Körperlumen nach der vorliegenden Erfindung kann der Stent in einer inerten Umgebung gelagert werden, die ihn vor einer Rekontamination oder vor Beschädigungen schützt, während er durch die Crimpvorrichtung im Durchmesser reduziert wird und in oder auf einem Katheter angeordnet wird. Insbesondere wird eine hydrophile Oberflächeneigenschaft des Stents während dem Crimpvorgang nicht verändert. Die Gefahr, dass bei der Implantation durch einen kontaminierten Stent Risiken für den Patienten entstehen wird dadurch deutlich reduziert.

Bei einer Ausführungsform der vorliegenden Erfindung kann als Schlauch eine Verpackung, in der der Stent inert gelagert ist, vorgesehen sein. Die Verpackung samt Stent kann durch eine Zuführung in die Bereitstellungseinrichtung eingeführt werden, bis die Elemente der Crimpvorrichtung den Stent von ausserhalb der Verpackung umfassen. Durch Betätigung des Aktivators wird der Stent innerhalb der Verpackung komprimiert, indem die Elemente von einer aufgeweiteten Position in eine geschlossene Position bewegt werden. Vorzugsweise weist die Verpackung dabei eine formbare Wandung auf. Beispielsweise ist sie als Beutel ausgebildet. Die Wandung kann auch derart flexibel sein, dass sie nach dem Komprimieren des Stents wieder in ihre Ausgangsform zurück springt.

Bei einer anderen Ausführungsform kommen zumindest die Elemente der Crimpvorrichtung, die beim Komprimieren des Stents unmittelbar auf den Stent wirken, innerhalb des Lagerraums und somit innerhalb der inerten Umhüllung des Stents zu liegen. Beispielweise können schwenkbare Segmente oder Backen, die mittels einer Welle bewegt werden, innerhalb des Lagerraums vorgesehen werden. In diesem Fall kann zwischen dem Stent und den Elementen eine Schutzhülle vorgesehen werden. Die Schutzhülle ist vorzugsweise aus inertem Material, wie z. B. Teflon bzw. ePTFE. Es ist auch möglich, dass die Elemente der Crimpvorrichtung zumindest in den Bereichen, an welchen sie den Stent berühren, eine inerte Oberfläche aufweisen. Die Oberfläche der Elemente kann hierfür z.B. mit Teflon beschichtet sein. Die Elemente können auch zumindest teilweise aus inertem Material bestehen, wie etwa Teflon bzw. ePTFE. Vorzugsweise weist die Oberfläche der Elemente eine hydrophile Eigenschaft. Hierfür können die Elemente z. B. gemeinsam mit dem Stent einer Reinigungsbehandlung, wie vorher beschreiben, unterzogen werden. Als hydrophile Eigenschaft der Oberfläche ist zu verstehen, dass die Oberfläche einen Kontaktwinkel kleiner als 90° aufweist. Der Grad der Hydrophilizität hängt dabei von der Art des Materials der Elemente ab.

Die Bereitstellungseinrichtung kann nicht nur zum Komprimieren des Stents dienen, sondern auch den komprimierten Stent in oder auf einem Katheter anbringen. Hierfür ist zumindest ein distales Ende eines Katheters innerhalb des Lagerraums zur Aufnahme des Stents in komprimiertem Zustand vorgesehen, wobei einem ballon-expandierenden bzw. einem selbst-expandierenden Stent komplementär ein Ballonkatheter bzw. ein Schlauchkatheter zugeordnet ist. Das proximale Ende des Katheters kann dabei aus dem Lagerraum durch einen Zugang herausragen. Der Katheter kann aber auch vollständig im Lagerraum untergebracht sein, beispielsweise wenn der Lagerraum durch eine Transportverpackung gegeben ist. Der unkomprimierte Stent kann bereits auf dem Katheter vormontiert, bzw. positioniert sein, wenn er in der Crimpvorrichtung zu liegen kommt.

Bei einer Ausführungsvariante besteht der Lagerraum aus einer Verpackung, die aus einem Behältnis mit einem Boden und einem Deckel besteht. Als Boden und Deckel sind dabei zwei sich gegenüberliegende Seiten, bzw. Wandbereiche, des Behältnisses zu verstehen. Der Boden und/oder der Deckel lassen sich entfernen. Der Boden und/oder der Deckel weisen einen zu öffnenden Zugang auf, so dass sich der Stent aus der Verpackung entnehmen lässt, bzw. der auf einem Katheter montierte Stent sich zusammen mit dem Katheter aus der Verpackung entnehmen lässt.

Der Katheter hat an seinem distalen Ende eine Spitze, und das der Spitze gegenüber liegende proximale Ende des Schafts des Katheters durchragt den Zugang nach ausserhalb der Verpackung.

Im Boden oder im Deckel ist ein Durchgang zum Durchlass einer Welle vorhanden, welche nach innen in die Verpackung zu den Backen einer integrierten Crimpvorrichtung und nach aussen zu einem Aktivator zur Betätigung der Crimpvorrichtung führt. Dem Durchgang gegenüberliegend im Deckel bzw. im Boden ist der zu öffnende Zugang vorhanden, der dem Durchlass eines Katheters dient. Axial durch die Crimpvorrichtung erstreckt sich ein Führungsdorn, welcher zur Stabilisierung und Positionierung nach dem vollständigen Einführen in ein Führungsdrahtlumen des Katheters bestimmt ist. Der zu öffnende Zugang ist vorteilhaft z.B. aus einer durchdringbaren Dichtung oder aus einem perforierbaren Material beschaffen. In der Verpackung erstrecken sich Stützelemente zur Fixierung des Stents und/oder des Katheters und/oder der Crimpvorrichtung. Die Bereitstellungseinrichtung kann die verschiedenen Vorrichtungen, die der Handhabung des Stents dienen, z. B. in einem Gehäuse unterbringen. Beispielweise kann die Behandlungsvorrichtung und die Crimpvorrichtung im Gehäuse angeordnet sein. Das Gehäuse weist eine Öffnung für die Zuführung, bzw. die Entnahme des Stents oder des Lagerraums mit dem Stent auf. Es können auch zwei Öffnungen vorgesehen sein, wovon eine als Zuführöffnung und eine als Entnahmeöffnung dient. Sofern der Lagerraum im Gehäuse integriert ist, ist im Gehäuse eine Vorrichtung zur Versorgung des Lagerraums mit der inerten Füllung vorgesehen. Die Versorgungsvorrichtung kann von ausserhalb des Gehäuses bedient werden, beispielweise mittels Zu- und Ableitungen für das inerte Medium. Ist der Lagerraum herausnehmbar im Gehäuse angeordnet, können Zu- und Ableitungen für das inerte Medium gemeinsam mit dem Lagerraum in das Gehäuse ein- und ausgeführt werden.

Nach der vorliegenden Erfindung wird ein Verfahren zum Bereitstellen eines Stents zur Implantation in ein Körperlumen vorgeschlagen, das für einen Stent vorgesehen ist, der ein proximales Ende und ein distales Ende aufweist, zwischen denen sich ein Stentlumen mit komprimierbarem Durchmesser erstreckt. Bei dem Bereitstellungsverfahren wird der Stent in einem Lagerraum bestehend aus einem inerten Medium, bzw. mit einer inerten Füllung, der eine Umhüllung für den Stent bildet, gelagert und mit einer Crimpvorrichtung mit beweglichen Elementen, die den Stent umfassen und von einer aufgeweiteten Position in eine geschlossene Position bewegt werden, komprimiert. Die Umhüllung ist als an einem Ende geschlossener Schlauch ausgebildet und seitlich komprimierbar ist. Dabei verbleibt der Stent in der inerten Umhüllung oder es wird eine Unterbrechung der inerten Umhüllung über eine Zeitspanne vorgesehen, in der keine signifikante Kontamination an einer Oberfläche des Stents, bzw. keine signifikante Erhöhung eines Kontaktwinkels eines Wassertropfens auf der Oberfläche des Stents erfolgt.
Dabei kann die Unterbrechung der inerten Umhüllung während des Komprimierens des Stents erfolgen. Beispielsweise kann ein Gasstrom eines inerten Gases, das den Lagerraum für den Stent bildet, kurze Zeit abgeschaltet werden. Oder ein mit einer inerten Flüssigkeit benetzter Stent kann für kurze Zeit trocken werden. Nach der Erfindung ist es auch möglich, dass das Komprimieren des Stents während der Unterbrechung erfolgt, solange die für das Komprimieren notwendige Zeit nicht länger ist, als die Zeitspanne der Unterbrechung in der keine signifikante Kontamination des Stents erfolgt. Dabei kann der Stent vor der Unterbrechung der inerten Umhüllung in einem ersten inerten Medium, bzw. einer ersten inerten Füllung, und nach der Unterbrechung der inerten Umhüllung in einem zweiten inerten Medium, bzw. einer zweiten inerten Füllung, gelagert werden. Der Stent kann also aus einer ersten inerten Umhüllung entnommen werden, von der Crimpvorrichtung komprimiert werden und anschliessend in eine zweite inerte Umhüllung eingebracht werden.

Vorzugsweise wird bei dem erfindungsgemässen Verfahren vor oder während der inerten Lagerung des Stents eine Reinigungsbehandlung durchgeführt, bei der die aus der Atmosphäre stammenden molekularen chemischen Verunreinigungen, vorrangig Kohlenwasserstoff-Verbindungen, auf der Oberfläche des Stents signifikant reduziert werden. Dadurch verringert sich der Kontaktwinkel als Mass der Hydrophilizität eines an der Oberfläche aufsitzenden Wassertropfens im Verhältnis zum Kontaktwinkel vor dieser Behandlung. Ferner wird der Stent vorzugsweise nach dem Komprimieren in einer Verpackung mit einer inerten Füllung gelagert. Letztlich ist es auch möglich nach dem Komprimieren eine Sterilisation des Stents durchzuführen, um u. a. Mikroorganismen abzutöten.

Nach dem Verfahren der vorliegenden Erfindung ist es besonders vorteilhaft, dass der Stent während der Abfolge der Verfahrensschritte der Reinigungsbehandlung, der Lagerung, des Komprimierens, des Verpackens und ggf. des Sterilisierens in der inerten Umhüllung verbleibt oder eine Unterbrechung der inerten Umhüllung über eine Zeitspanne vorgesehen ist, in der keine signifikante Kontamination, bzw. keine signifikante Erhöhung des Kontaktwinkels eines Wassertropfens auf der Stentoberfläche erfolgt. Es besteht somit eine im Wesentlichen sichere Umgebung, in der der Stent keiner Rekontaminaton ausgesetzt ist, während alle Schritte zur Bereitstellung des Stent für eine Implantation vom Zeitpunkt der Reinigungsbehandlung bis hin zur Verpackung durchgeführt werden. Sollte eine Unterbrechung dieser sicheren Umgebung vorgesehen werden, ist diese von derart kurzer Dauer, dass keine signifikante Rekontamination erfolgt und der klinische Erfolg bei der Implantation eines solchen Stents nicht in Frage gestellt ist.

Vorteilhafterweise kann der Stent in einer Bereitstellungseinrichtung, wie vorher beschrieben, komprimiert werden. Der Stent wird dann während des Komprimierens nicht oder nur unwesentlich der Umgebungsatmosphäre oder anderen kontaminierenden Stoffen ausgesetzt. Eine Verunreinigung der Stentoberfläche beim Crimpvorgang kann dadurch vermieden werden.

Bei einer Variante des erfindungsgemässen Verfahrens kann der Stent in einer Verpackung vorgesehen sein, die als Lagerraum dient und eine inerte Füllung aufweist. Die Verpackung mit dem Stent wird in die Bereitstellungseinrichtung mit der Crimpvorrichtung eingeführt, d. h. sie wird zwischen die Elemente der Crimpvorrichtung eingesetzt, die für das Komprimieren des Stents radial beweglich sind. Das Komprimieren des Stents muss daher nicht in einem Reinraum erfolgen. Der Stent kann beim Hersteller oder auch vor Ort in den normalen Räumlichkeiten eines Spitals für die Implantation bereit gemacht werden.

Bei einer anderen Variante des Verfahrens wird die Crimpvorrichtung zumindest teilweise innerhalb des Lagerraums bestehend aus einem inerten Medium, bzw. mit einer inerten Füllung gelagert oder in diesen eingeführt und von ausserhalb des Lagerraums betätigt. Wird als Lagerraum beispielsweise ein Behälter vorgesehen, dessen inerte Füllung durch Wasser oder ein Gas gegeben ist, das schwerer als Luft ist, können z. B. die beweglichen Elemente der Crimpvorrichtung durch eine obere Öffnung im Behälter in den Behälter eingeführt werden. Der Stent kann somit in der inerten Umhüllung im Lagerraum verbleiben und muss nicht aus dieser geschützten Umgebung entnommen werden, um einen Crimpprozess zu durchlaufen.

Eine Reinigungsbehandlung, wie vorher erwähnt, kann z. B. materialabtragend erfolgen, nämlich z.B. mittels Sputtern als Beschuss mit Ionen, Elektroerosion, Elektropolieren, Plasmaaktivieren, Laserablation, mechanisch abrasivem Verfahren, Trockenätzen oder nass-chemisch Ätzen. Alternativ hat die Behandlung der Oberfläche zur Reduktion der chemischen Verunreinigungen eine unveränderte Topographie der Oberfläche als Ergebnis, wobei auch hier die Behandlung z.B. mittels Sputtern als Beschuss mit Ionen, Elektroerosion, Elektropolieren, Plasmaaktivieren, Laserablation, mechanisch abrasivem Verfahren, Trockenätzen oder nass-chemisch Ätzen erfolgen kann. Eine nicht material-abtragende Behandlung, z.B. mittels Ultraschall, UV-Licht oder Ozon oder einer daraus gebildeten Kombinationsbehandlung, kann ebenfalls zu einer unveränderten Oberflächentopographie führen. Gleichermassen ist hierfür ein Ätzmedium geeignet, welches das Stentmaterial selbst nicht angreift.

Ferner ist es vorteilhaft, dass Verunreinigungen auch von der Oberfläche von Elementen der Crimpvorrichtung, die zum Komprimieren des Stents am Stent angreifen, durch eine Reinigungsbehandlung signifikant reduziert werden. Es können die gleichen Reinigungsverfahren wie für den Stent verwendet werden. Besonders vorteilhaft werden die Elemente der Crimpvorrichtung gemeinsam mit dem Stent gereinigt.

Bei einer bevorzugten Ausführung des erfindungsgemässen Verfahrens wird der Stent im inerten Lagerraum auf oder in einem Katheter komprimiert angeordnet, so dass der Stent aus dem Lagerraum entnommen werden kann, ohne dabei einer Rekontamination ausgesetzt zu werden. Hierfür wird zumindest ein distales Ende eines Katheters im Lagerraum vorgesehen. Bei einem ballon-expandierenden Stent, wird der Stent auf einen Ballon am distalen Ende des Katheters komprimiert. Bei einem selbst-expandierenden Stent wird der Stent von der Crimpvorrichtung komprimiert und anschliessend in das distale Ende eines Schlauchkatheters eingeführt, bzw. der Schlauchkatheter über den komprimierten Stent geschoben. Es ist auch möglich den Stent gemeinsam mit dem Katheter mit Hilfe eines Tranfergefässes aus dem Lagerraum zu entnehmen, wie vorher beschrieben.

Im Falle eines selbst-expandierenden Stents ist es auch möglich, den Stent nach dem Crimpvorgang zu kühlen, um die Beibehaltung des komprimierten Zustands des Stents zu unterstützen. Dies ist z. B. bei Nickel-Titan-Stents sinnvoll. Andererseits können auch ballon-expandierende Stents während dem Komprimieren temperiert werden, um die Haftung auf dem Ballonkatheter zu erhöhen. Eine geeignete Temperatur ist dabei vom Material des Stents abhängig. In der Bereitstellungseinrichtung ist hierfür eine Temperiervorrichtung vorgesehen.

Bei dem Verfahren nach der Erfindung finden alle Schritte zur Bereitstellung des Stents zur Implantation in ein Körpervolumen nach dessen Herstellung in einer kontrollierten Umgebung statt. Vorzugsweise wird der Stent nach der Reinigung der Oberfläche, insbesondere zur Erzeugung einer hydrophilen Oberfläche, in den Lagerraum eingebracht oder befindet sich bei der Reinigung bereits im Lagerraum, wird im Lagerraum komprimiert und anschliessend auf oder in dem Katheter angeordnet. Zur Implantation wird der Katheter, bzw. dessen distales Ende mit dem Stent, aus dem Lagerraum entnommen und in den Körper des Patienten eingeführt. Erst am Behandlungsort im Körper wird der Stent freigesetzt. Ab der Reinigungsbehandlung befindet sich der Stent somit zumindest die überwiegende Zeit in der kontrollierten Umgebung und eine erneute signifikante Verunreinigung kann ausgeschlossen werden. Wie bereits beschrieben, wird die Reinheit des Stents durch kurze Unterbrechungen der inerten Umhüllung nicht beeinträchtigt. Derartige Unterbrechungen können beispielsweise beim Transfer des Stents von der Reinigungsbehandlung in die Bereitstellungseinrichtung oder von der Bereitstellungseinrichtung in eine Versandverpackung erfolgen. Weiter kann eine solche Unterbrechung auch während eines Arbeitsschrittes zur Bereitstellung des Stents, z. B. während des Komprimierens erfolgen, solange dabei keine signifikante Rekontamination eintritt.

Es ist somit grundsätzlich auch möglich, zwischen oder während den Arbeitsvorgängen zur Bereitstellung des Stents zur Implantation die inerte Umgebung zu wechseln, solange dabei sichergestellt ist, dass keine signifikante Rekontamination erfolgt. Beispielsweise kann der Stent in einem flüssigen Medium inert gelagert sein, während er einer Reinigungsbehandlung unterzogen wird, und anschliessend aus dem flüssigen Medium in eine Verpackung mit einer gasförmigen inerten Füllung transferiert werden. Bei der Entnahme aus dem flüssigen Medium kann ein Flüssigkeitsfilm auf der Oberfläche des Stents verbleiben und ihn gegen Verunreinigung schützen bis er in die neue inerte Umgebung der Verpackung eingebracht ist. Mit der Verpackung kann der Stent in die Bereitstellungseinrichtung mit der Crimpvorrichtung eingeführt und darin komprimiert werden.

### KURZE BESCHREIBUNG DER ZEICHNUNGEN

Bevorzugte Ausführungsformen der Erfindung werden im Folgenden anhand der Zeichnungen dargestellt, die lediglich zur Erläuterung dienen und nicht einschränkend auszulegen sind. Aus den Zeichnungen offenbar werdende Merkmale der Erfindung sollen einzeln und in jeder Kombination als zur Offenbarung der Erfindung gehörend betrachtet werden. In den Zeichnungen zeigen:
- Figur 1A:: einen Stent, ballon- oder selbst-expandierend, im nicht-gecrimpten Zustand;
- Figur 1B:: Lagerraum in Form einer Verpackung mit darin in einer Inertfüllung gelagertem Stent gemäss Figur 1A;
- Figur 2A:: Einrichtung zur Bereitstellung eines Stents zur Implantation in ein Körperlumen mit Zugang in einen Lagerraum und darin integrierter offener Crimpvorrichtung;
- Figur 2B:: die Crimpvorrichtung aus Figur 2A im offenen Zustand;
- Figur 2C:: die Crimpvorrichtung aus Figur 2A im geschlossenen Zustand;
- Figur 3A:: Einrichtung zur Bereitstellung eines Stents zur Implantation in ein Körperlumen nach einer ersten Ausführungsform mit einem ballon-expandierenden Stent und einer innerhalb des Lagerraums angeordneten Crimpvorrichtung in geöffnetem Zustand ;
- Figur 3B:: Einrichtung nach der ersten Ausführungsform mit der Crimpvorrichtung in geschlossenem Zustand;
- Figur 4:: Verpackung mit darin in einer Inertfüllung gelagertem ballon-expandierenden Stent, auf einem Dilatationskatheter im gecrimpten Zustand;
- Figur 5A:: Einrichtung zur Bereitstellung eines Stents zur Implantation in ein Körperlumen nach einer zweiten Ausführungsform mit einem selbst-expandierenden Stent, einem Katheter und einer innerhalb des Lagerraums angeordneten Crimpvorrichtung in geöffnetem Zustand;
- Figur 5B:: Einrichtung nach der zweiten Ausführungsform mit der Crimpvorrichtung in geschlossenem Zustand;
- Figur 5C:: Einrichtung nach der zweiten Ausführungsform mit dem Stent in komprimiertem Zustand und geöffneter Crimvorrichtung;
- Figur 5D:: Einrichtung nach der zweiten Ausführungsform mit einem Aussenschlauch des Katheters, der partiell über den gecrimpten Stent geschoben ist;
- Figur 5E:: Einrichtung nach der zweiten Ausführungsform mit einem Aussenschlauch des Katheters, der vollständig über den gecrimpten Stent geschoben ist;

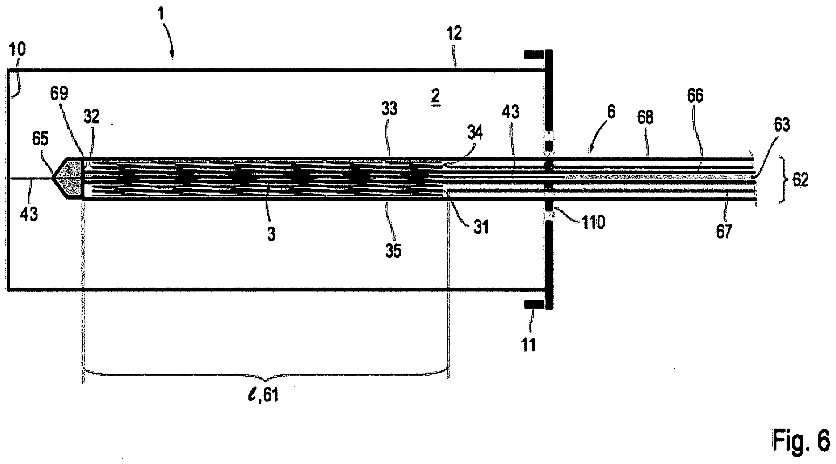

- Figur 6:: Lagerraum mit darin in einer Inertfüllung gelagertem selbst-expandierenden Stent, auf einem Katheter montiert, in gecrimptem Zustand ;
- Figur 7A:: erfindungsgemässe Einrichtung nach einer dritten Ausführungsform mit einem ballon-expandierenden Stent und einer ausserhalb des Lagerraums angeordneten Crimpvorrichtung in geöffnetem Zustand;
- Figur 7B:: erfindungsgemässe Einrichtung nach der dritten Ausführungsform mit der Crimpvorrichtung in geschlossenem Zustand ;
- Figur 8A:: erfindungsgemässe Einrichtung nach einer vierten Ausfiihrungsform mit einem selbst-expandierendem Stent und einer ausserhalb des Lagerraums angeordneten Crimpvorrichtung in geöffnetem Zustand;
- Figur 8B :: erfindungsgemässe Einrichtung nach der vierten Ausführungsform mit der Crimpvorrichtung in geschlossenem Zustand;
- Figur 8C:: erfindungsgemässe Einrichtung nach der vierten Ausführungsform mit der Crimpvorrichung in geöffnetem Zustand und dem Stent in komprimiertem Zustand;
- Figur 9A:: Einrichtung nach einer fünften Ausführungsform mit einer Schutzhülle, einem ballon-expandierendem Stent und geöffneter Crimpvorrichtung;
- Figur 9B:: Einrichtung nach der fünften Ausführungsform mit geschlossener Crimpvorrichtung und
- Figur 10:: Einrichtung nach der fünften Ausführungsform mit einem selbst-expandierendem Stent.

In den dargestellten Ausührungsbeispielen werden gleiche Bauteile mit gleichen Bezugszeichen benannt. Für die gesamte weitere Beschreibung gilt folgende Festlegung. Sind in einer Figur zum Zweck zeichnerischer Eindeutigkeit Bezugsziffern enthalten, aber im unmittelbar zugehörigen Beschreibungstext nicht erläutert, so wird auf deren Erwähnung in vorangehenden oder nachfolgenden Figurenbeschreibungen Bezug genommen. Im Interesse der Übersichtlichkeit wird auf die wiederholte Bezeichnung von Bauteilen in weiteren Figuren zumeist verzichtet, sofern zeichnerisch eindeutig erkennbar ist, dass es sich um "wiederkehrende" Bauteile handelt.

### Figur 1A :

Der dargestellte Stent 3 ist von herkömmlicher Materialbeschaffenheit und konstruktivem Aufbau; er könnte ballon- oder selbst-expandierend sein. Der Stent 3 hat die Länge 1, welche sich zwischen dem proximalen Ende 31 und dem distalen Ende 32 erstreckt. Im nicht-gecrimpten Zustand nimmt der Stent 3 den Durchmesser d ein, so dass die Stege 33, welche die Oberfläche 35 besitzen, gitterförmig, weiträumig von einander beabstandet sind. Durch den röhrchenförmigen Stent 3 verläuft das im Prinzip zylindrische Stentlumen 34. Es ist möglich, dass der Stent 3 eine Beschichtung trägt, insbesondere eine Beschichtung mit aktiven Substanzen, die in den Körper eines Patienten eingebracht werden sollen.

### Figur 1B:

Der Stent 3 steckt in einem Lagerraum in Form einer Verpackung 1, der in eine Bereitstellungseinrichtung eingesetzt werden kann. Der Stent 3 wird hierbei von einer in der Verpackung 1 angeordneten Halterung 13 fixiert, die zunächst ein erstes Stützelement 131 umfasst, an dem das proximale Ende 31 ansteht. Vom zweiten Stützelement 132 wird das distale Ende 32 gefasst. Die Verpackung 1 umfasst zunächst das Behältnis 12 mit dem Boden 10 und ist am zum Boden 10 gegen überliegenden Ende mit dem Deckel 11 verschlossen. Behältnis 12, Boden 10 und Deckel 11 können einteilig ausgebildet sein, vorzugsweise ist zumindest der Deckel 11 abnehmbar, bzw. kann zum Öffnen des Behältnisses 12 aufgeklappt oder geöffnet werden. Das erste Stützelement 131 erstreckt sich trennwandartig über die Querschnittsfläche des Behältnisses 12 und ist dem Deckel 11 zugewandt, wobei ein drittes Stützelement 133 axial den Deckel 11 mit dem ersten Stützelement 131 verbindet. Das zweite Stützelement 132 erstreckt sich ebenfalls trennwandartig über die Querschnittsfläche des Behältnisses 12, ist aber dem Boden 10 zugewandt. In dem Lagerraum 1 befindet sich eine inerte Füllung 2, welche die Oberfläche 35 des Stent 3 schützt. Die dem Stent 3 zugewandten Innenflächen des Lagerraums 1 sind inert.

Durch vorgängige Behandlung der Oberfläche 35 wurde deren hydrophile Eigenschaft erhöht. Die aus der Atmosphäre stammenden molekularen chemischen Verunreinigungen auf der Oberfläche 35 - hauptsächlich Kohlenwasserstoff-Verbindungen - wurden signifikant reduziert, wodurch sich der Kontaktwinkel als Mass der Hydrophilizität eines an der Oberfläche 35 aufsitzenden Wassertropfens verringert.

Die Reduktion der chemischen Verunreinigungen auf der Oberfläche 35 lässt sich auch durch Materialabtrag erzielen. Hierfür bieten sich Sputtern als Beschuss mit Ionen, Elektroerosion oder - polieren, Plasmaaktivieren, Laserablation, mechanisch abrasive Verfahren, Trockenätzen oder nass-chemisch Ätzen an. Alternativ wird die Reduktion der chemischen Verunreinigungen auf der Oberfläche 35 durch eine die Topographie der Oberfläche 35 unverändernde Behandlung erzielt. Hierfür kommt die Behandlung mittels Ultraschall, UV-Licht, Ozon oder eine daraus gebildete Kombinationsbehandlung in Betracht. Gleichermassen eignet sich die Behandlung mit einem Ätzmedium, welches das Stentmaterial selbst nicht angreift, z. B. eine Säurebehandlung der Oberfläche. Bewährt hat sich 95% - 97% Schwefelsäure auf Kobalt-Chrom-Legierung und auf Nickel-Titan-Legierung.

Es ist auch möglich, die Oberflächenbehandlung innerhalb des Lagerraums in Form der Verpackung 1 durchzuführen. In diesem Fall ist ein nich Material abtragendes Reinigungsverfahren vorzuziehen.

### Figuren 2A bis 2C :

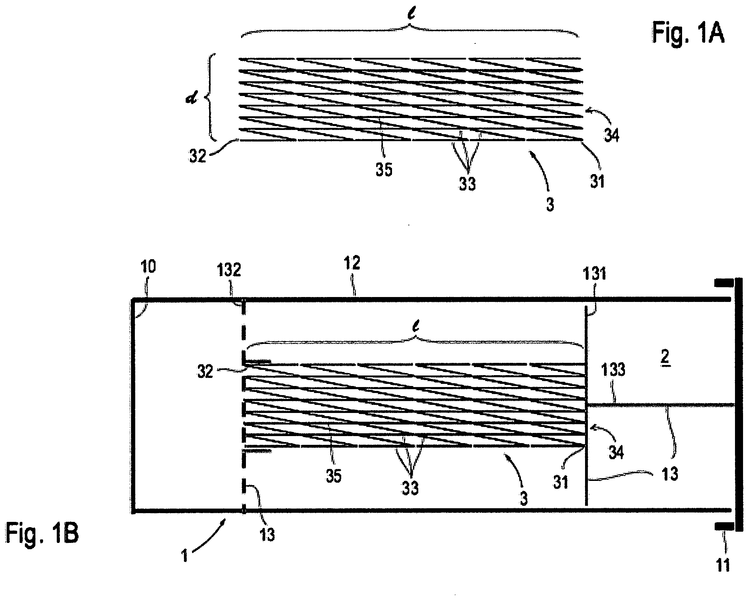

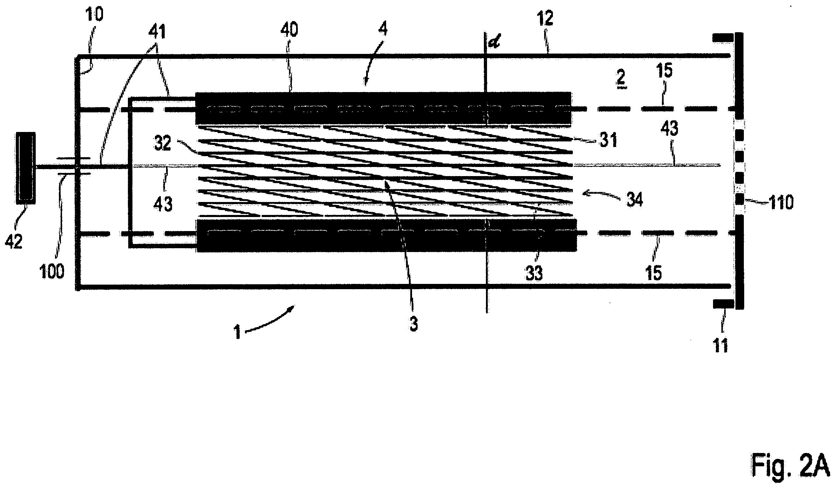

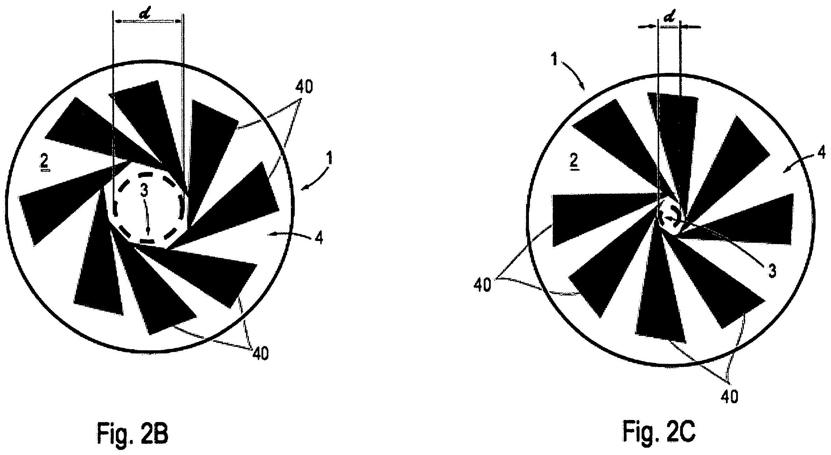

Diese Figurengruppe illustriert schematisch die Funktion einer Einrichtung zur Bereitstellung eines Stents zur Implantation in ein Köperlumen. Die Einrichtung umfasst den Lagerraum in Form der Verpackung 1, eine Crimpvorrichtung 4 mit Crimpelementen in Form von Backen 40 und einen Aktivator 42 zur Betätigung der Crimpvorrichtung. In dem Lagerraum in Form der Verpackung 1 sind der Stent 3 und die Backen 40 der Crimpvorrichtung 4 in einer inerten Füllung 2 gelagert. Die Crimpvorrichtung 4 ist zunächst offen, so dass deren Backen 40 eine aufgeweitete Position einnehmen und dabei den in der Verpackung 1 steckenden, expandierten Stent 3 umfassen (s. Figuren 2A, 2B). Die Vorbehandlung des Stents 3 geschieht, wie bereits zur Figur 1B beschrieben. Die Verpackung 1 beinhaltet wiederum die inerte Füllung 2 und die Innenwandung der Verpackung ist inert. Die Backen 40 sitzen auf einer Welle 41, die axial durch einen Durchgang 100 im Lagerraum nach aussen zu einem betätigbaren Aktivator 42 führt. Das Behältnis 12 wird von sich axial zwischen dem Boden 10 und dem Deckel 11 erstreckenden Achsen 15 durchragt. Zentrisch durch das Behältnis 12 verläuft ein zur Crimpvorrichtung 4 gehörender Führungsdorn 43, der innerhalb des Behältnisses 12 vor einem perforierbaren Zugang 110 am Lagerraum endet. Bei geschlossener Crimpvorrichtung 4 sind die Backen 40 radial verengt, so dass der Stent 3 im Durchmesser d komprimiert ist (siehe Figur 2C).

### Figuren 3A und 3B:

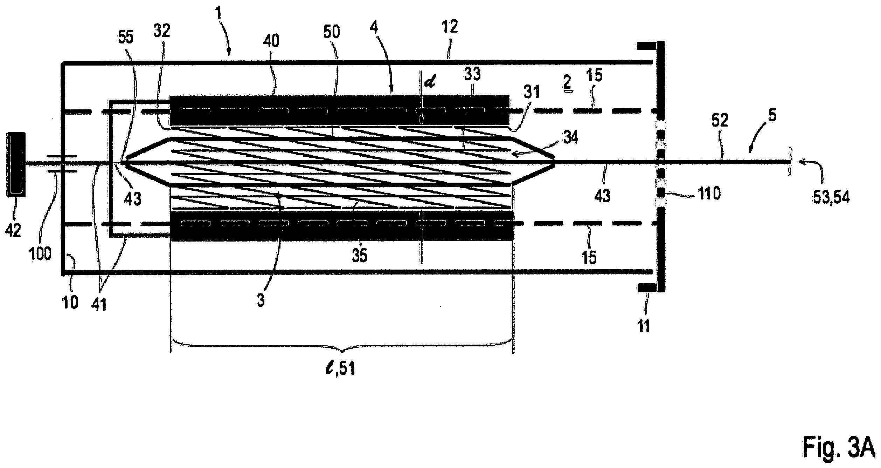

Dieses Figurenpaar zeigt eine erste Ausführungsform einer Einrichtung mit einem ballon-expandierendem Stent und einer innerhalb des Lagerraums angeordneten Crimpvorrichtung. Der Stent 3 wurde einer Vorbehandlung zur Erhöhung der Hydrophilizität der Oberfläche 35 unterzogen, wie bei Figur 1B dargelegt. Vorausgesetzt werden wiederum eine inerte Füllung des Lagerraums in Form der Verpackung 1 sowie inerte Eigenschaft deren Innenwandung. Die Backen 40 der Crimpvorrichtung 4 sind zunächst offen (s. Figur 3A). Durch den perforierbaren Zugang 110 im Lagerraum hat man mit der Spitze 55 voran den auf dem Schaft 52 angeordneten Ballon 50 des Katheters 5 in das Stentlumen 34 eingeführt. Hierbei ist der Führungsdorn 43 in das Führungsdrahtlumen 53 des Schafts 52 eingedrungen. Der Schaft 52 hat ferner das kanalartige Dilatationslumen 54, über welches von aussen, während der Operation, der Ballon 50 durch Auffüllen von innen - z. B. mittels physiologischer Salzlösung - zur Expansion gebracht wird und damit den Stent 3 von innen aufweitet. Der Ballon 50 sitzt mit seinem Stentbereich 51 im Stentlumen 34, so dass der Stentbereich 51 zumindest im Prinzip die gesamte Stentlänge 1 durchragt, während die sich zuspitzenden Enden des Ballons 50 aus dem proximalen Ende 31 und dem distalen Ende 32 des Stent 3 herausragen.

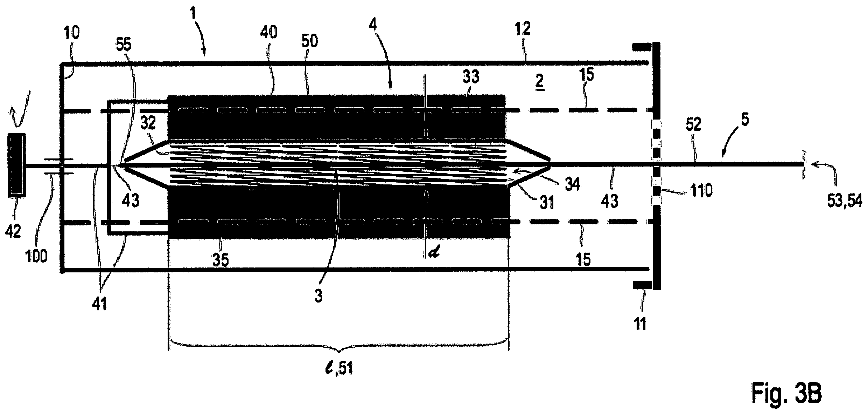

Nach Betätigung des Aktivators 42 durch Drehen, z. B. per Hand, gelangt die Crimpvorrichtung 4 in den geschlossenen Zustand, so dass der Stent 3 in seinem Durchmesser d zusammengedrückt wird (siehe Figur 3B). Bei dem nun verengten Stentdurchmesser d und den komprimierten Backen 40 der Crimpvorrichtung 4 bleibt der Ballon 50 mit seinem Stentbereich 51 innerhalb des Stentlumen 34 in unveränderter axialer Position.

### Figur 4 :

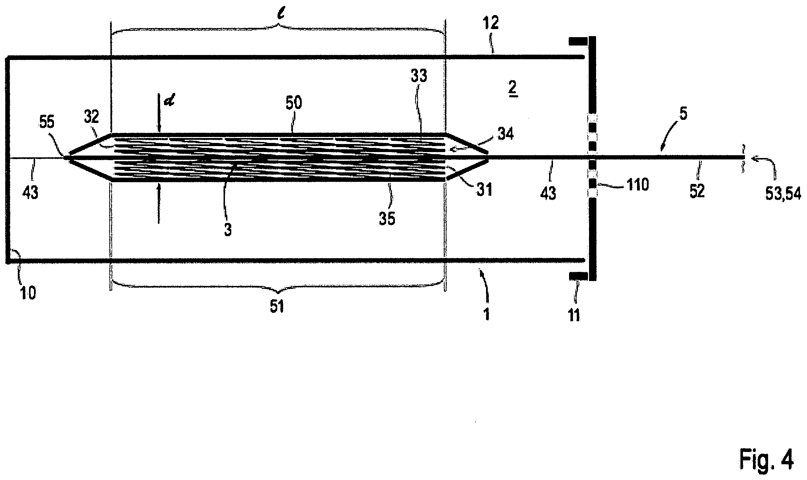

In Figur 4 ist die Verpackung 1 gezeigt, aus der die Crimpvorrichtung 4 entfernt wurde, bzw. der Lagerraum aus der Bereitstellungseinrichtung entnommen wurde. In der Verpackung 1 kann nun der ballon-expandierende Stent 3 auf dem Ballon 50 eines Dilatationskatheters 5 im gecrimpten Zustand aufbewahrt werden. Dabei ist der Stentdurchmesser d verengt und die Stege 33 liegen aneinander gedrängt. Erneut erstreckt sich der Stentbereich 51 des Ballons 50 zumindest im Prinzip über die Stentlänge 1. Der Führungsdorn 43, der sich vom Boden 10 erstreckt, ist in das Führungsdrahtlumen 53 des Schafts 52 eingedrungen. Die Spitze 55 kommt nahe dem Boden 10 zu liegen. Das Innere der Verpackung 1 ist mit der inerten Füllung 2 versehen, welche die Oberfläche 35 des gemäss Beschreibung zu Figur 1B vorbehandelten Stent 3 schützt. Ferner wird vorausgesetzt, dass die Innenwandung der Verpackung 1 inert ist. Durch den perforierbaren Zugang 110 im Lagerraum lässt sich der Dilatationskatheter 5 samt gecrimpten Stent 3 und Ballon 50 aus der Verpackung 1 herausziehen. Beim Einbringen des Lagerraums mit dem Stent 3 in die Bereitstellungseinrichtung zum Komprimieren des Stents können z. B. auch die Elemente der Crimpvorrichtung durch den Zugang 110 in den Lagerraum eingeführt werden. Alternativ können die Elemente der Crimpvorrichtung auch in den Lagerraum eingebracht werden, indem der Deckel oder der Boden abgenommen und die Elemente in die Verpackung eingeführt werden.

### Figuren 5A bis 5E:

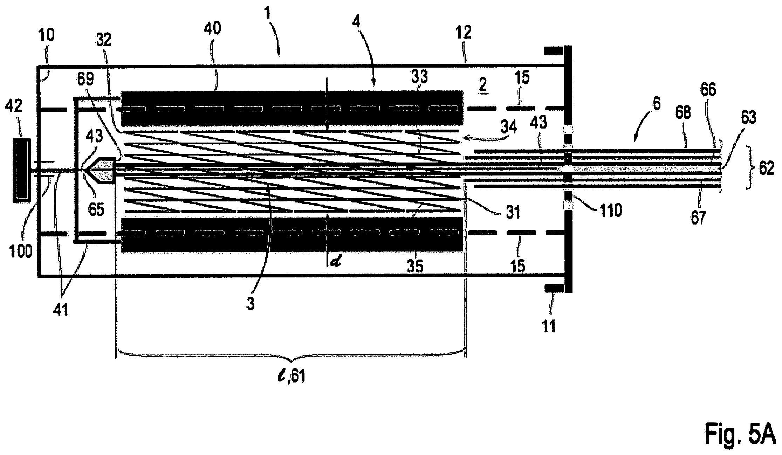

In den Figur 5A bis 5E ist eine zweite Ausführungsform einer Einrichtung zur Bereitstellung eines Stents 3 zum Implantieren in ein Körperlumen gezeigt, in deren Lagerraum in Form der Verpackung 1 ein selbst-expandierenden Stent sowie die Crimpelemente in Form von Backen 40 der Crimpvorrichtung 4 angeordnet sind. Ein Schlauchkatheter 6 ist mit seinem distalen Ende in den Lagerraum eingeführt. Zur Crimpvorrichtung 4 gehören wiederum die sich durch den Durchgang 100 im Boden 10 zum Aktivator 42 hin erstreckende Welle 41 und der die Verpackung 1 axial durchragende Führungsdorn 43. Die Verpackung 1 enthält die inerte Füllung 2 und die Verpackungsinnenwandung ist inert. Innerhalb der Verpackung 1 liegen erneut die Achsen 15. Die Oberfläche 35 des Stent 3 ist zur Erhöhung der Hydrophilizität vorbehandelt, wie bei Figur 1B erläutert.

### Figur 5A (Ausgangssituation):

Die Backen 40 der Crimpvorrichtung 4 sind offen, der Stent 3 befindet sich folglich im ungecrimpten Zustand und der Innenschlauch 66 des Schlauchkatheters 6 ist durch den perforierbaren Zugang 110 im Deckel 11 des Lagerraums und das Stentlumen 34 soweit eingeschoben, dass die Spitze 65 dem Boden 10 zugewandt aus dem Stent 3 herausragt. Der Führungsdorn 43 ist axial in das Führungsdrahtlumen 63 des Schafts 62 eingedrungen. Der Stützschlauch 67 und der Aussenschlauch 68 sind ebenfalls durch den perforierbaren Zugang 110 eingeschoben, deren freie Enden stehen jedoch vor dem proximalen Ende 31 des Stents 3. Zwischen dem freien Ende des Stützschlauchs 67 und dem Anschlag 69 an der Spitze 65 erstreckt sich der Stentbereich 61, der die Stentlänge 1 aufnehmen kann.

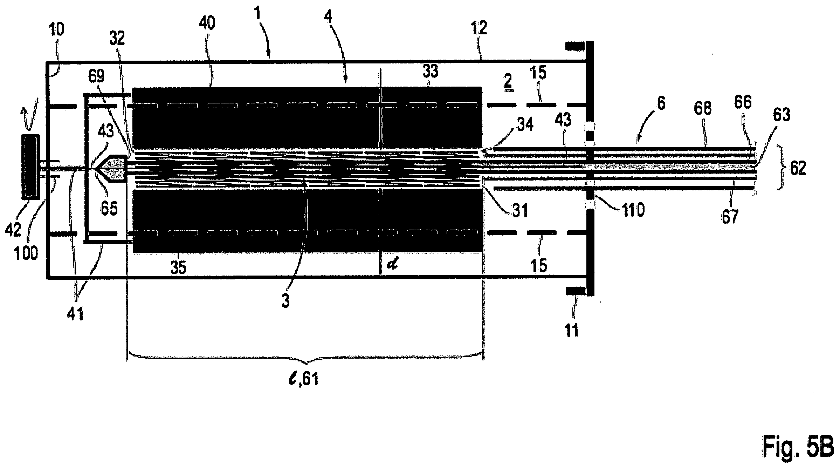

### Figur 5B (1. Fortschritt):

Die Backen 40 der Crimpvorrichtung 4 sind nun geschlossen, so dass die Stege 33 des Stents 3 zusammengedrängt liegen und der Stentdurchmesser d verengt ist. Die Betätigung der Crimpvorrichtung 4 erfolgte durch Drehen am Aktivator 42, der ausserhalb des Lagerraums angeordnet ist. Der Schlauchkatheter 6 mit der Spitze 65, dem Innenschlauch 66, dem Stützschlauch 67 und dem Aussenschlauch 68 ist in der Positionierung unverändert. Im gecrimpten Zustand kann der Stent 3 heruntergekühlt werden, um das bei Unterschreiten einer definierten Temperatur selbst-expandierende Verhalten auszuschalten. Zur Kühlung können z. B. ein Kühlspray oder Kühlelemente, wie etwa Peltier-Elemente, verwendet werden. Bei der Wahl des inerten Mediums, bzw. der inerten Füllung ist dabei darauf zu achten, dass das Medium oder die Füllung durch die Kühlung keine Zustandsänderung durchläuft, wie etwa gefriert.

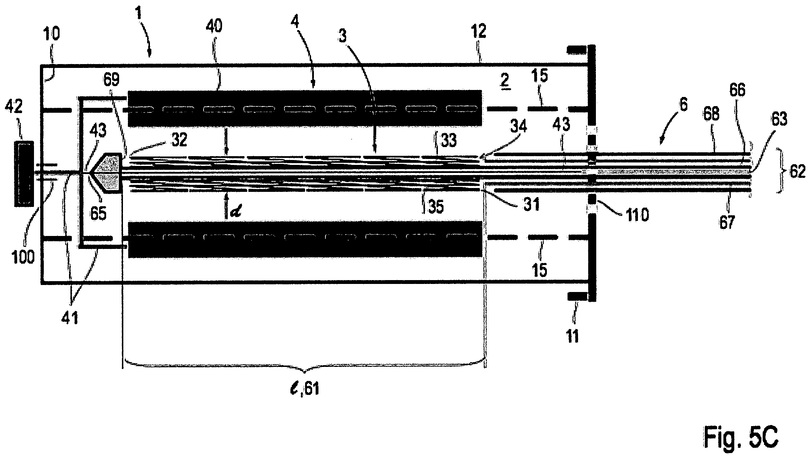

### Figur 5C (2. Fortschritt):

Die Backen 40 der Crimpvorrichtung 4 werden geöffnet, wobei der selbst-expandierende Stent 3 im gecrimpten Zustand mit dem verengten Stentdurchmesser d und den verdichteten Stegen 33 durch die zuvor abgesenkte Temperatur verharrt.

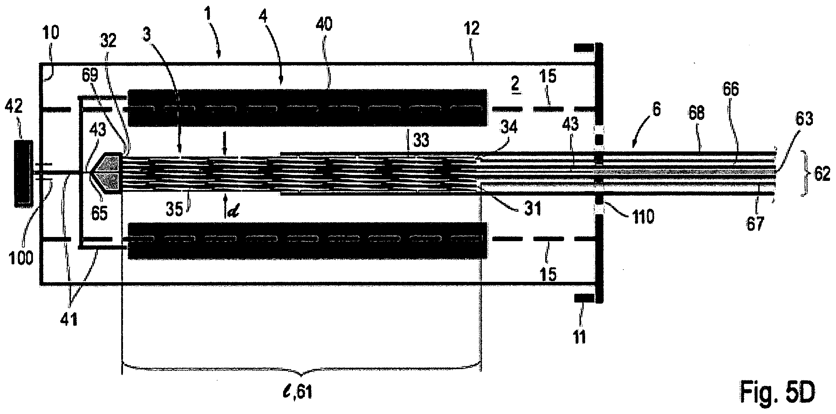

### Figur 5D (3. Fortschritt):

Der im gecrimpten Zustand mit dem verengten Stentdurchmesser d verharrende Stent 3 erlaubt vom proximalen Ende 31 ein sukzessives Aufschieben des Aussenschlauches 68 auf den Stent 3 in Richtung des distalen Endes 32. Der Stützschlauch 67 und die am Innenschlauch 66 angeordnete Spitze 65 bleiben in unveränderter Position. Durch das Vorschieben des Aussenschlauches 68 wird der Stent 3 in gleiche Richtung mitbewegt, wobei der Anschlag 69 das weitere Vorschieben des Stents 3 stoppt.

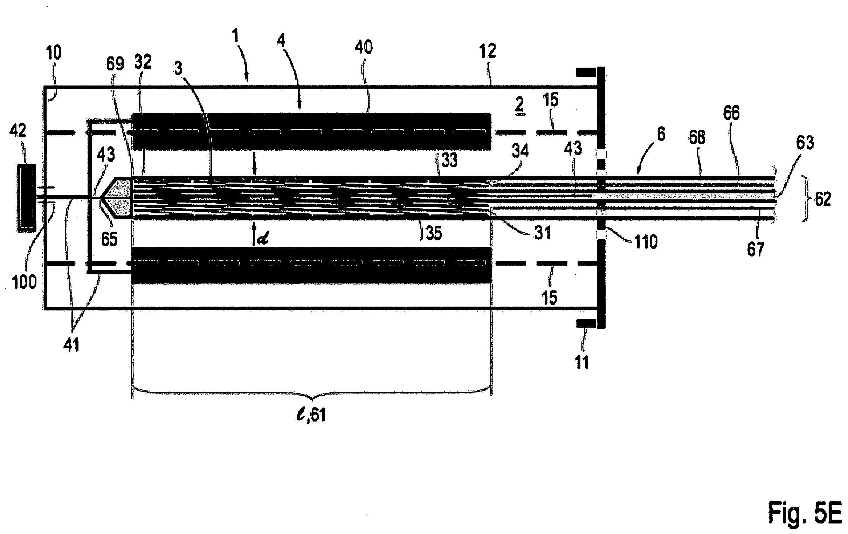

### Figur 5E (4. Fortschritt):

Der Aussenschlauch 68 ist soweit über den gecrimpten Stent 3 aufgeschoben, bis er hinter der Spitze 65 auf den Anschlag 69 trifft und folglich nun den gesamten Stentbereich 61 überdeckt. Der Stent 3 ist während der Arbeitsschritte des Komprimierens und des Aufnehmens in den Katheter in der inerten Füllung 2 im Lagerraum der Bereitstellungseinrichtung gelagert, so dass keinerlei unerwünschte Kontamination beim Überführen aus der Crimpvorrichtung in den Katheter erfolgen kann. Zur Implantation des Stents zieht man den Schlauchkatheter 6 mit dem darin gefassten, gecrimpten Stent 3 durch den perforierbaren Zugang 110 aus der Verpackung 1 heraus, um den so vorbereiteten Stent 3 dem Patienten an der zuvor bestimmten Körperstelle zu applizieren.

### Figur 6:

Grundsätzlich ist es möglich, die Backen 40 aus dem Lagerraum zu entfernen und den Stent im Lagerraum auf zu bewahren, so dass dieser als Verpackung für den Stent dient. Der Führungsdorn 43 ist in das Führungsdrahtlumen 63 eingefahren. Der Schaft 62 mit Aussenschlauch 68, Stützschlauch 67 und Innenschlauch 66 durchragen den perforierbaren Zugang 110 im Lagerraum nach aussen. Der Aussenschlauch 68 steht am Anschlag 69 der Spitze 65 an und übergreift somit den gesamten Stentbereich 61. Das freie Ende des Stützschlauchs 67 befindet sich vor dem proximalen Ende 31 des Stents 3. Die weitere Handhabung erfolgt wie im Anschluss an Figur 5E.

### Figuren 7A und 7B:

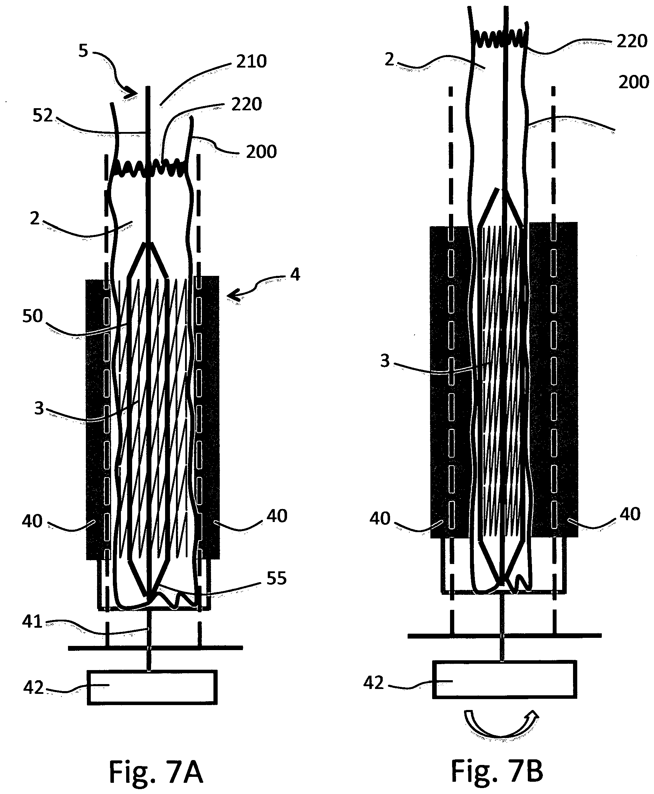

In den Figuren 7A und 7B ist eine dritte Ausführungsform einer Bereitstellungseinrichtung nach der vorliegenden Erfindung gezeigt, bei der die Crimpvorrichtung 4 vollständig ausserhalb eines Lagerraums 200 angeordnet ist. Der Lagerraum 200 ist als an einem Ende geschlossener Schlauch ausgebildet, der seitlich komprimierbar ist, so dass sich der Durchmesser des Schlauchs verringern kann. Am gegenüberliegenden Ende weist der Schlauch eine Öffnung 210 auf. Der Schlauch kann aus inertem Material gefertigt sein oder nur eine inerte Innenoberfläche aufweisen. Der Innenraum des Schlauchs 200 ist mit einem inerten flüssigen Medium 2 gefüllt. Es ist darauf zu achten, dass der Füllstand des Mediums 2 in aufgeweitetem Zustand des Schlauchs derart gewählt wird, dass das Medium 2 auch in komprimiertem Zustand des Schlauchs, in dem sich das Innenvolumen gegenüber dem aufgeweiteten Zustand verringert, nicht aus dem Schlauch entweicht. Im Lagerraum 200 ist ein ballon-expandierender Stent 3 und zugehöriger Ballonkatheter 5, wie er ausführlich bei den Figuren 3A und 3B beschrieben ist, vorgesehen. Der Füllstand 220 des inerten Medium 2 im Schlauch 200 ist so hoch, dass es den Stent 3 und den Ballon 50 zumindest die überwiegende Zeit vollständig umgibt, so dass der Stent im Lagerraum inert gelagert ist.

Die Crimpvorrichtung 4 umfasst die Backen 40 und die Welle 41. Ein Aktivator 42 dient zur Betätigung der Crimpvorrichtung 4. Die Öffnung der Crimpvorrichtung zwischen den Crimpelementen der Bereitstellungseinrichtung ist vertikal angeordnet, so dass der Schlauch 200 vertikal mit der Öffnung 210 nach oben in die Crimpvorrichtung 4 der Bereitstellungseinrichtung eingeführt werden kann und die Backen 40 den Stent 3 umfassen. Es ist grundsätzlich auch möglich, eine horizontale Anordnung der Crimpelemente und des darin eingebrachten Stents zu wählen, solange die Öffnung 210 nach oben weist und sichergestellt ist, dass die inerte Füllung nicht aus dem Schlauch austritt, während der Stent komprimiert und somit das Volumen des Schlauches verkleinert wird.

In Figur 7A ist der Stent in unkomprimiertem Zustand mittels des Schlauchs 200 mit der Öffnung 210 nach oben in die Crimpvorrichtung eingeführt worden. Der Stent 3 ist dabei in der inerten Füllung 2 gelagert und vor einer Rekontamination geschützt.

In Figur 7B ist die Crimpvorrichtung 4 mit verengten Backen 40 gezeigt, so dass der Lagerraum und der Stent im Lagerraum komprimiert sind. Hierfür wurde der Aktivator 42 betätigt, z. B. gedreht, um die Backen 40 nach innen in den freien Raum um die Achse der Crimpvorrichtung 4 zu bewegen, so dass sie am Aussenumfang des Stents angreifen und diesen zur Achse hin komprimieren. Dabei wird der Stent auf den Ballon aufgepresst, wie es bei den Figuren 3A und 3B beschrieben ist. Der Füllstand 220 des inerten Mediums 2 im Schlauch steigt durch die Kompression des Schlauchs an.

Nach dem Crimpvorgang können die Backen 40 mittels dem Aktivator 42 wieder geöffnet werden und der Schlauchlagerraum 200 aus der Bereitstellungseinrichtung entnommen werden. Der Füllstand 220 sinkt wieder auf seinen anfänglichen Wert. Der Stent 3 und der Ballon 50 des Katheters 5 bleiben während dem gesamten Ablauf angefangen vom Einführen des Lagerraums in die Bereitstellungseinrichtung, über den Crimpvorgang bis zur Entnahme aus der Bereitstellungseinrichtung innerhalb des inerten Mediums gelagert. Der Schlauch kann nach der Entnahme aus der Bereitstellungseinrichtung an der Öffnung 210 verschlossen werden, so dass der Schlauch als Transportverpackung dienen kann.

### Figuren 8A bis 8C:

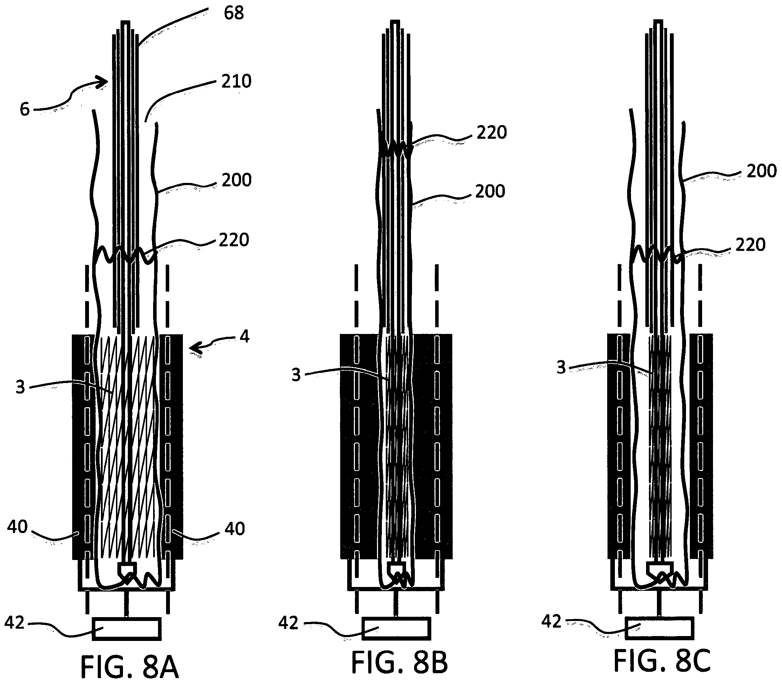

In den Figuren 8A bis 8C ist eine vierte Ausführungsform einer Bereitstellungseinrichtung nach der vorliegenden Erfindung gezeigt, bei der die Crimpvorrichtung 4 ebenfalls vollständig ausserhalb eines Lagerraums 200 angeordnet ist. Der Lagerraum 200 ist als Schlauch ausgebildet und mit einem inerten Medium 2 gefüllt analog zur Ausführungsform nach den Figuren 7A und 7B. In dem Schlauch ist ein selbst-expandierender Stent 3 gelagert und das distale Ende eines SchlauchKatheters 6 ist durch die Öffnung 210 eingeführt. Der Schlauch-Katheter ist im wesentlichen entsprechend der Ausführungsform nach den Figuren 5A bis 5E ausgebildet. Der Katheter wird so weit in den Lagerraum, d. h. den Schlauch 200, eingeführt, dass die Enden des Aussenschlauchs 68 und des Stützschlauchs 67 in das inerte Medium 2 hinein ragen und zwar sowohl bei einem Füllstand 220 mit geöffneten Backen 40 als auch mit geschlossenen Backen 40 der Crimpvorrichtung 4.

Die Crimpvorrichtung ist vertikal in der Bereitstellungseinrichtung ausgerichtet und wird über den Aktivator 42 betätigt.

In Figur 8A ist die Bereitstellungseinrichtung mit geöffneten Backen 40 der Crimpvorrichtung 4 gezeigt, wobei der Stent von den Backen 40 umfasst wird. In Figur 8B wurde der Aktivator 42 betätigt, so dass die Backen 40 auf den Stent 3 einwirken und diesen komprimieren. Der Stent kann nun gekühlt werden, z. B. durch Kühlung des inerten Mediums oder in anderer Weise, um das selbst-expandierende Verhalten auszuschalten, wie bei Figur 5B beschrieben. Anschliessend kann die Crimpvorrichtung geöffnet werden, wie in Figur 8C gezeigt. Der Aussenschlauch 68 kann über den Stent geschoben werden, wie bei den Figuren 5D und 5E erläutert, wobei der Stent wiederum zwischen dem Anschlag 69 und dem Stützschlauch 67 gehalten wird. Sobald der Aussenschlauch 68 den gesamten Stentbereich 61 überdeckt und an dem Anschlag 69 anschlägt, ist der Stent innerhalb des Katheter inert gelagert und kann aus dem Schlauch 200 entnommen werden, ohne dass eine erneute Verunreinigung erfolgen kann. Der Stent und der Katheter können aber auch gemeinsam mit dem Schlauch 200 aus der Bereitstellungseinrichtung entnommen werden, so dass der Schlauch 200 nach Verschliessen der Öffnung 210 wiederum als Transportverpackung dienen kann.

### Figuren 9A und 9B:

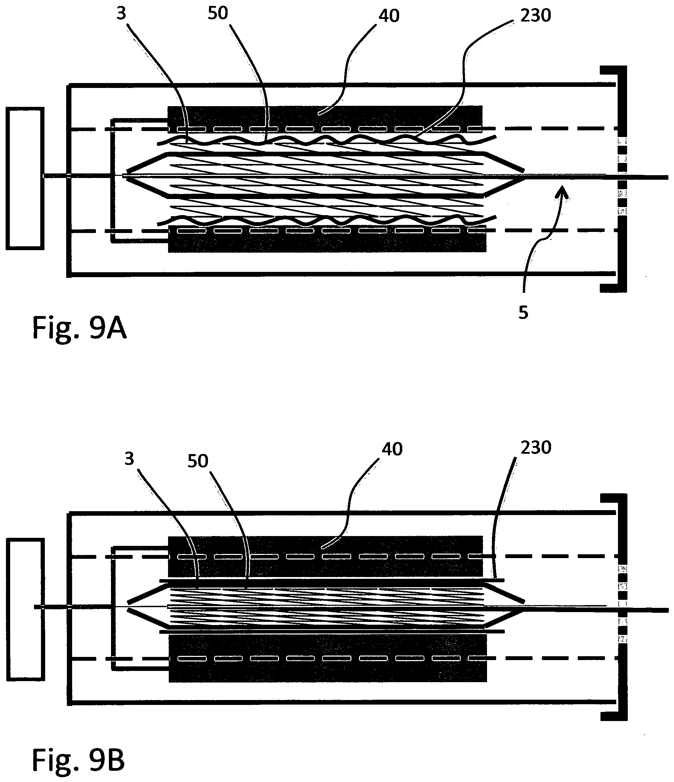

In den Figuren 9A und 9B ist eine Bereitstellungseinrichtung nach einer fünften Ausführungsform gezeigt. Die Bereitstellungseinrichtung entspricht im Wesentlichen derjenigen aus den Figuren 3A und 3B, bei der sich die Crimpbacken 40 der Crimpvorrichtung 4 innerhalb des Lagerraums und somit innerhalb der inerten Füllung 2 befinden. Zwischen den Backen 40 und dem Stent 3 ist eine Schutzhülle 230 über die gesamte Länge 1 des Stents vorgesehen, die den Stent 3 umgibt. Die Backen 40 kommen somit beim Komprimieren des Stents nicht unmittelbar auf der Oberfläche 35 des Stents zu liegen. Die Schutzhülle 230 kann beim Einbringen des Stents in den Lagerraum gemeinsam mit diesem eingeführt werden. Sie kann aber auch fest an den Elementen der Crimpvorrichtung angebracht sein, bzw. austauschbar an diesen angeordnet werden.

In Figur 9A ist die Bereitstellungseinrichtung mit geöffneter Crimpvorrichtung 4 gezeigt, wobei der Stent in einem expandierten Zustand ist. In Figur 9B ist die Crimpvorrichtung 4 geschlossen und der Stent wurde auf den Ballon 50 aufgecrimpt.

### Figur 10:

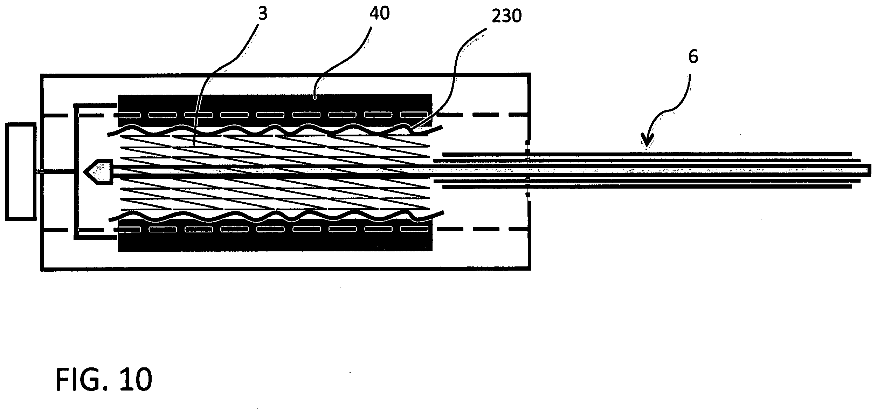

In Figur 10 ist eine Bereitstellungseinrichtung nach der fünften Ausführungsform unter Verwendung eines selbst-expandierenden Stents 3 und eines Schlauchkatheters 6 gezeigt, bei der wiederum eine Schutzhülle 230 den Stent 3 umgibt. Das Komprimieren des Stents erfolgt analog der Vorgehensweise, wie sie in den Figuren 5A bis 5E beschrieben ist. Sobald die Backen 40 nach dem Crimpvorgang wieder geöffnet werden, weitet sich auch die Schutzfolie 230 wieder so weit auf, dass der Aussenschlauch 68 zwischen der Schutzhülle 230 und der Stentoberfläche 35 hindurchgeschoben werden kann, bis der Stent im Katheter aufgenommen ist. Die Schutzhülle 230 verbleibt dabei ausserhalb des Katheters.

In den dargestellten Ausführungsformen kommt eine Crimpvorrichtung mit Backenelementen, die zum Komprimieren auf den Stent wirken, zum Einsatz. Grundsätzlich sind aber auch andere Crimpvorrichtung zur Verwendung in der erfindungsgemässen Bereitstellungseinrichtung geeignet, wie sie z. B. bei der Beschreibung zum Stand der Technik dargestellt wurden.

Zur Durchführung des Verfahrens ist es auch möglich, den Stent vollständig aus seiner inerten Umhüllung zu entnehmen, einer Crimpvorrichtung zu zu führen, die den Stent ausserhalb der inerten Umhüllung komprimiert, solange die Zeitspanne, in welcher der Stent nicht von der inerten Umhüllung geschützt wird, keine signifikante Rekontamination zulässt. Ein derartiges Vorgehen ist grundsätzlich auch mit einer erfindungsgemässen Bereitstellungseinrichtung möglich.

**BEZUGSZEICHENLISTE**

| | | | |
|---|---|---|---|
| 1 | Lagerraum | 50 | Ballon |
| 2 | Füllung | 51 | Stentbereich |
| 3 | Stent | 52 | Schaft |
| 4 | Crimpvorrichtung | 53 | Führungsdrahtlumen |
| 5 | Ballon-Katheter | 54 | Dilatationslumen |
| 6 | Schlauch-Katheter | 55 | Spitze |
| 10 | Boden | 61 | Stentbereich |
| 11 | Deckel | 62 | Schaft |
| 12 | Behältnis | 63 | Führungsdrahtlumen |
| 13 | Halterung | 65 | Spitze |
| 15 | Achse | 66 | Innenschlauch |
| 31 | proximales Ende | 67 | Stützschlauch |
| 32 | distales Ende | 68 | Aussenschlauch |
| 33 | Stege | 69 | Anschlag |
| 34 | Stentlumen | 200 | Lagerraum |
| 35 | Oberfläche | 210 | Öffnung |
| 40 | Backen | 220 | Füllstand |
| 41 | Welle | 230 | Schutzhülle |
| 42 | Aktivator | | |
| 43 | Führungsdorn | | |
| | | l | Stentlänge |
| | | d | Stentdurchmesser |

## Patentansprüche

1. Einrichtung zur Bereitstellung eines Stents zur Implantation in ein Körperlumen, wobei der Stent (3) ein proximales Ende (31) und ein distales Ende (32) aufweist, zwischen denen sich ein Stentlumen mit komprimierbarem Durchmesser d erstreckt,
- die eine Crimpvorrichtung (4) mit Elementen (40), die um eine Achse angeordnet und zumindest zum Teil relativ zu einander radial zur Achse beweglich sind, und
- einen Aktivator (42) zur Betätigung der Crimpvorrichtung (4) aufweist,
- wobei die Elemente (40) den Stent (3) umfassen und mittels des Aktivators (42) von einer aufgeweiteten Position, in der der Stent (3) ungecrimpt ist, in eine geschlossene Position, in der der Stent (3) im Durchmesser d komprimiert ist, radial beweglich sind,
**dadurch gekennzeichnet, dass** ein Lagerraum (200) mit einer inerten Füllung vorgesehen ist, der als an einem Ende geschlossener Schlauch ausgebildet und seitlich komprimierbar ist, wobei ein hydrophilisierter Stent (3) in dem Lagerraum (200) in die Bereitstellungseinrichtung einsetzbar ist, und der Lagerraum (200) eine Umhüllung bildet, während die Elemente (40) den Stent (3) umfassen und von der aufgeweiteten Position in die geschlossene Position beweglich sind, so dass der Stent zumindest die überwiegende Zeit inert gelagert ist.

2. Einrichtung nach Anspruch 1, **dadurch gekennzeichnet, dass** die Crimpvorrichtung (4) ausserhalb des Lagerraums des Stents (3) angeordnet ist.

3. Einrichtung nach Anspruch 1 oder 2, **dadurch gekennzeichnet, dass** als Schlauch eine Verpackung, in der der Stent (3) inert gelagert ist, vorgesehen ist.

4. Einrichtung nach Anspruch 3, **dadurch gekennzeichnet, dass** die Verpackung eine formbare Wandung aufweist.

5. Einrichtung nach Anspruch 3 und 4, **dadurch gekennzeichnet, dass** eine Zuführung zum Zuführen der Verpackung mit dem Stent zwischen die Elemente (40) der Crimpvorrichtung (4) vorgesehen ist.

6. Einrichtung nach einem der vorhergehenden Ansprüche, **dadurch gekennzeichnet, dass** der Lagerraum (200) mit einer inerten Füllung gefüllt ist, die schwerer als Luft ist.

7. Einrichtung nach einem der vorhergehenden Ansprüche, **dadurch gekennzeichnet, dass** der Stent (3) derart überwiegend inert gelagert ist, dass eine Unterbrechung der inerten Umhüllung über eine Zeitspanne vorgesehen sein kann, in der keine signifikante Rekontamination der Stentoberfläche (35) erfolgt.

8. Einrichtung nach Anspruch 7, **dadurch gekennzeichnet, dass** die Zeitspanne der Unterbrechung der inerten Umhüllung ca. 15 Minuten nicht überschreitet.

9. Einrichtung nach einem der vorhergehenden Ansprüche, **dadurch gekennzeichnet, dass** zumindest ein distales Ende eines Katheters innerhalb des Lagerraums (200) zur Aufnahme des Stents in komprimiertem Zustand vorgesehen ist, wobei einem ballon-expandierenden bzw. einem selbst-expandierenden Stent (3) komplementär ein Ballonkatheter (5) bzw. ein Schlauchkatheter (6) zugeordnet ist.

10. Einrichtung nach Anspruch 9, **dadurch gekennzeichnet, dass** das proximale Ende des Katheters aus dem Lagerraum durch einen Zugang herausragt.

11. Verfahren zum Bereitstellen eines Stents zur Implantation in ein Körperlumen, wobei der Stent (3) ein proximales Ende (31) und ein distales Ende (32) aufweist, zwischen denen sich ein Stentlumen mit komprimierbarem Durchmesser d erstreckt, umfassend:
- Lagerung des Stents (3) in einem Lagerraum (200) mit einer inerten Füllung, der als an einem Ende geschlossener Schlauch ausgebildet und seitlich komprimierbar ist und eine Umhüllung für den Stent (3) bildet,
- Reinigungsbehandlung vor oder während der inerten Lagerung des Stents (3), wodurch sich der Kontaktwinkel als Mass der Hydrophilizität eines an der Oberfläche (35) aufsitzenden Wassertropfens im Verhältnis zum Kontaktwinkel vor dieser Behandlung verringert, und
- Komprimieren des Stents (3) mit einer Crimpvorrichtung (4) mit beweglichen Elementen (40), die den Stent (3) umfassen und von einer aufgeweiteten Position in eine geschlossene Position bewegt werden, wobei der Stent (3) in der inerten Umhüllung verbleibt.

12. Verfahren nach Anspruch 11, **dadurch gekennzeichnet, dass** bei der Reinigungsbehandlung die aus der Atmosphäre stammenden molekularen chemischen Verunreinigungen, vorrangig Kohenwasserstoff-Verbindungen, auf der Oberfläche (35) des Stents (3) signifikant reduziert werden.

13. Verfahren nach einem der Ansprüche 11 bis 12, **dadurch gekennzeichnet, dass** der Stent (3) nach dem Komprimieren in einer Verpackung mit einer inerten Füllung gelagert wird.

14. Verfahren nach einem der Ansprüche 11 bis 13, **dadurch gekennzeichnet, dass** nach dem Komprimieren eine Sterilisation des Stents (3) erfolgt.

15. Verfahren nach den Ansprüchen 11 bis 14, **dadurch gekennzeichnet, dass** der Stent während der Abfolge der Verfahrensschritte der Reinigungsbehandlung, der Lagerung, des Komprimierens, des Verpackens und ggf. des Sterilisierens in der inerten Umhüllung des Lagerraums (200) verbleibt oder eine Unterbrechung der inerten Umhüllung über eine Zeitspanne vorgesehen ist, in der keine signifikante Kontamination an einer Oberfläche (35) des Stents (3), bzw. keine signifikante Erhöhung eines Kontaktwinkels eines Wassertropfens auf der Oberfläche (35) des Stents (3) erfolgt.

16. Verfahren nach einem der Ansprüche 11 bis 15, **dadurch gekennzeichnet, dass** der Lagerraum durch eine Verpackung (200) des Stents (3) gegeben ist, die in die Crimpvorrichtung (4) eingeführt wird.

17. Verfahren nach Anspruch 15, **dadurch gekennzeichnet, dass** der Stent vorder Unterbrechung der inerten Umhüllung in einem ersten inerten Medium, bzw. einer ersten inerten Füllung, und nach der Unterbrechung der inerten Umhüllung in einem zweiten inerten Medium, bzw. einer zweiten inerten Füllung, gelagert wird.

18. Verfahren nach einem der Ansprüche 11 bis 17, **dadurch gekennzeichnet, dass** der Stent (3) während der inerten Lagerung an einem distalen Ende eines Katheters angeordnet wird, wobei bei einem ballon-expandierenden Stent, der Stent auf einen Ballon am distalen Ende des Katheters komprimiert wird, und bei einem selbst-expandierenden Stent der Stent von der Crimpvorrichtung komprimiert und anschliessend in das distale Ende eines Schlauchkatheters (6) eingeführt wird.

## Claims

1. Arrangement for providing a stent for implantation into a body lumen, the stent (3) having a proximal end (31) and a distal end (32) between which a stent lumen having a compressible diameter d extends, which arrangement comprises
- a crimping device (4) having elements (40) which are arranged around an axis and are at least partly movable relative to one another radially with respect to the axis, and
- an activator (42) for actuating the crimping device (4),
- wherein the elements (40) encompass the stent (3) and are radially movable by means of the activator (42) from an expanded position, in which the stent (3) is uncrimped, into a closed position, in which the stent (3) is compressed in diameter d,
**characterized in that** a storage space (200) comprising an inert filling is provided, which storage space (200) is designed as a tubing closed at one end and is laterally compressible, wherein a hydrophilized stent (3) is insertable in the storage space (200) into the providing arrangement, and the storage space (200) forms a casing, while the elements (40) encompass the stent (3) and can be moved from the expanded position into the closed position such that the stent is stored in an inert manner for at least a predominant time.

2. Arrangement according to claim 1, **characterized in that** the crimping device (4) is arranged outside the storage space of the stent (3).

3. Arrangement according to either claim 1 or claim 2, **characterized in that** a packaging in which the stent (3) is stored in an inert manner is provided as the tubing.

4. Arrangement according to claim 3, **characterized in that** the packaging has a shapeable wall.

5. Arrangement according to claim 3 and claim 4, **characterized in that** a feed is provided for feeding the packaging together with the stent between the elements (40) of the crimping device (4).

6. Arrangement according to any of the preceding claims, **characterized in that** the storage space (200) is filled with an inert filling which is heavier than air.

7. Arrangement according to any of the preceding claims, **characterized in that** the stent (3) is stored in a predominantly inert manner such that the inert casing can be interrupted over a period of time in which there is no significant recontamination of the stent surface (35).

8. Arrangement according to claim 7, **characterized in that** the period of time of the interruption of the inert casing does not exceed approximately 15 minutes.

9. Arrangement according to any of the preceding claims, **characterized in that** at least a distal end of a catheter is provided within the storage space (200) for receiving the stent in a compressed state, wherein a balloon-expanding or a self-expanding stent (3) is associated in a complementary manner with a balloon catheter (5) or a tube catheter (6).

10. Arrangement according to claim 9, **characterized in that** the proximal end of the catheter protrudes out of the storage space through an access.

11. Method for providing a stent for implantation into a body lumen, wherein the stent (3) has a proximal end (31) and a distal end (32) between which a stent lumen having a compressible diameter d extends, the method comprising:
- storing the stent (3) in a storage space (200) comprising an inert filling, which storage space is designed as a tubing closed at one end and is laterally compressible and forms a casing for the stent (3),
- performing a cleaning treatment before or during inert storage of the stent (3), as a result of which cleaning treatment, as a measure of hydrophilicity, the contact angle of a drop of water sitting on the surface (35) is reduced relative to the contact angle before this treatment, and
- compressing the stent (3) by means of a crimping device (4) comprising movable elements (40) which encompass the stent (3) and are moved from an expanded position into a closed position, wherein the stent (3) remains in the inert casing.

12. Method according to claim 11, **characterized in that**, during the cleaning treatment, the molecular chemical impurities originating from the atmosphere, primarily hydrocarbon compounds, on the surface (35) of the stent (3) are significantly reduced.

13. Method according to claim 11 or claim 12, **characterized in that**, after compression, the stent (3) is stored in a packaging comprising an inert filling.

14. Method according to any of claims 11 to 13, **characterized in that**, after compression, the stent (3) is sterilized.

15. Method according to claims 11 to 14, **characterized in that** the stent remains in the inert casing of the storage space (200) during the sequence of method steps of the cleaning treatment, storage, compression, packaging and possibly sterilization, or an interruption of the inert casing is provided over a period of time in which there is no significant contamination on a surface (35) of the stent (3), or no significant increase in a contact angle of a drop of water on the surface (35) of the stent (3).

16. Method according to any of claims 11 to 15, **characterized in that** the storage space is provided by a packaging (200) of the stent (3), which packaging is introduced into the crimping device (4).

17. Method according to claim 15, **characterized in that** the stent, before the inert casing is interrupted, is stored in a first inert medium or a first inert filling, and after the inert casing is interrupted, is stored in a second inert medium or a second inert filling.

18. Method according to any of claims 11 to 17, **characterized in that**, during inert storage, the stent (3) is arranged at a distal end of a catheter, the stent, in the case of a balloon-expanding stent, being compressed on a balloon at the distal end of the catheter, and, in the case of a self-expanding stent, being compressed by the crimping device and subsequently introduced into the distal end of the tube catheter (6).

## Revendications

1. Dispositif de mise en œuvre d'un stent pour l'implantation dans une lumière corporelle, le stent (3) présentant une extrémité proximale (31) et une extrémité distale (32) entre lesquelles s'étend une lumière de stent de diamètre d compressible,
- un dispositif de sertissage (4) pourvu d'éléments (40) disposés autour d'un axe et au moins partiellement mobiles l'un par rapport à l'autre de façon radiale par rapport à l'axe, et
- un actionneur (42) destiné à l'actionnement du dispositif de sertissage (4),
- les éléments (40) englobant le stent (3) et pouvant être déplacés de façon radiale au moyen de l'actionneur (42) d'une position ouverte, dans laquelle le stent (3) n'est pas serti, à une position fermée, dans laquelle le diamètre d du stent (3) est comprimé,
**caractérisé en ce qu'**un espace de stockage (200) renfermant un matériau de remplissage inerte est prévu, lequel espace de stockage est réalisé sous la forme d'un tube fermé à une extrémité et est compressible de façon latérale, un stent hydrophilisé (3) dans l'espace de stockage (200) pouvant être inséré dans le dispositif de mise œuvre,
et l'espace de stockage (200) formant une enveloppe, tandis que les éléments (40) englobent le stent (3) et peuvent être déplacés de la position ouverte à la position fermée, de sorte que le stent soit logé de façon inerte au moins la plupart du temps.

2. Dispositif selon la revendication 1, **caractérisé en ce que** le dispositif de sertissage (4) est disposé à l'extérieur de l'espace de stockage du stent (3).

3. Dispositif selon la revendication 1 ou 2, **caractérisé en ce qu'**un emballage, dans lequel le stent (3) est stocké inerte, est mis en œuvre sous forme de tube.

4. Dispositif selon la revendication 3, **caractérisé en ce que** l'emballage présente une paroi pouvant être moulée.

5. Dispositif selon les revendications 3 et 4, **caractérisé en ce qu'**un dispositif d'amenée est prévu pour amener le stent dans l'emballage entre les éléments (40) du dispositif de sertissage (4).

6. Dispositif selon l'une des revendications précédentes, **caractérisé en ce que** l'espace de stockage (200) est rempli d'un matériau de remplissage inerte qui est plus lourd que l'air.

7. Dispositif selon l'une des revendications précédentes, **caractérisé en ce que** le stent (3) est essentiellement inerte de telle manière qu'une interruption de l'enveloppe inerte peut être prévue pendant une période durant laquelle aucune recontamination significative de la surface du stent (35) ne se produit.

8. Dispositif selon la revendication 7, **caractérisé en ce que** la période d'interruption de l'enveloppe inerte n'excède pas environ 15 minutes.

9. Dispositif selon l'une des revendications précédentes, **caractérisé en ce qu'**au moins une extrémité distale d'un cathéter est prévue dans l'espace de stockage (200) pour recevoir le stent à l'état comprimé, dans lequel un cathéter à ballonnet (5) ou un cathéter tubulaire (6) est associé de façon complémentaire à un stent (3) expansible à ballonnet ou auto-expansible.

10. Dispositif selon la revendication 9, **caractérisé en ce que** l'extrémité proximale du cathéter fait saillie de l'espace de stockage par un accès.

11. Procédé de mise en œuvre d'un stent pour l'implantation dans une lumière corporelle, le stent (3) présentant une extrémité proximale (31) et une extrémité distale (32) entre lesquelles s'étend une lumière de stent de diamètre d compressible, comprenant :
- le stockage du stent (3) dans un espace de stockage (200) pourvu d'un matériau de remplissage inerte qui est réalisé sous la forme d'un tube fermé à une extrémité et compressible de façon latérale et qui forme une enveloppe pour le stent (3),
- le traitement de nettoyage avant ou pendant le stockage inerte du stent (3), de sorte que l'angle de contact, comme mesure de l'hydrophilicité d'une goutte d'eau sur la surface (35), soit réduit par rapport à l'angle de contact avant ledit traitement, et
- la compression du stent (3) à l'aide d'un dispositif de sertissage (4) pourvu d'éléments mobiles (40) qui englobent le stent (3) et sont déplacés d'une position ouverte à une position fermée, le stent (3) restant dans l'enveloppe inerte.

12. Procédé selon la revendication 11, **caractérisé en ce que**, lors du traitement de nettoyage, les impuretés chimiques moléculaires, principalement des composés d'hydrocarbures, provenant de l'atmosphère sont considérablement réduites sur la surface (35) du stent (3).

13. Procédé selon l'une des revendications 11 à 12, **caractérisé en ce que** le stent (3) est logé dans un emballage pourvu d'un matériau de remplissage inerte après compression.

14. Procédé selon l'une des revendications 11 à 13, **caractérisé en ce que**, après compression, la stérilisation du stent (3) est réalisée.

15. Procédé selon les revendications 11 à 14, **caractérisé en ce que** le stent reste dans l'enveloppe inerte de l'espace de stockage (200) pendant la succession des étapes de traitement de nettoyage, de stockage, de compression, d'emballage et éventuellement de stérilisation, ou **en ce qu'**une interruption de l'enveloppe inerte est prévue pendant une période durant laquelle aucune contamination significative à la surface (35) du stent (3) ou aucune augmentation significative de l'angle de contact d'une goutte d'eau sur la surface (35) du stent (3) ne se produit.

16. Procédé selon l'une des revendications 11 à 15, **caractérisé en ce que** l'espace de stockage est assuré par un emballage (200) du stent (3) qui est inséré dans le dispositif de sertissage (4).

17. Procédé selon la revendication 15, **caractérisé en ce que** le stent est logé dans un premier fluide inerte, ou un premier matériau de remplissage inerte avant l'interruption de l'enveloppe inerte, et dans un second fluide inerte, ou un second matériau de remplissage inerte après l'interruption de l'enveloppe inerte.

18. Procédé selon l'une des revendications 11 à 17, **caractérisé en ce que** le stent (3) est disposé à une extrémité distale d'un cathéter pendant le stockage inerte, dans lequel, dans le cas d'un stent expansible à ballonnet, le stent est comprimé sur un ballonnet à l'extrémité distale du cathéter, et dans le cas d'un stent auto-expansible, le stent est comprimé par le dispositif de sertissage et ensuite inséré dans l'extrémité distale d'un cathéter tubulaire (6).
